Europäisches Patentamt

European Patent Office

Office européen des brevets

(19)

(11) **EP 1 364 639 A2**

(12) **EUROPEAN PATENT APPLICATION**

(43) Date of publication:
26.11.2003 Bulletin 2003/48

(51) Int Cl.⁷: **A61K 7/00**

(21) Application number: 03011595.0

(22) Date of filing: 22.05.2003

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: 31.05.2002 US 384975 P
23.05.2002 US 383057 P

(71) Applicants:
• **Schott Glas**
55122 Mainz (DE)
• **ENGELHARD CORPORATION**
Iselin, New Jersey 08830-0770 (US)

(72) Inventors:
• **Zimmer, José, Dr.**
55218 Ingelheim (DE)
• **Fechner, Jörg, Dr.**
55118 Mainz (DE)
• **Uzunian, Gabe**
10562 Ossining, New York (US)
• **Aucar, Betty F.**
10562 Ossining, New York (US)

(74) Representative: **Dr. Weitzel & Partner**
**Friedenstrasse 10**
**89522 Heidenheim (DE)**

(54) **Formulation of cosmetic products with glass powder**

(57) The invention concerns a Cosmetic formulation comprising 99,9 weight -% - 70 weigth-% of a basic cosmetic fomulation selected from the group of transparent pearly lotion, gel cologne with sparkles, pearlescent waterproof sun creme, bronze self tanning cream, sunscreen creme, pearl bronze/copper suntan cream, Icy gel toothpaste, face mask for oily skin, moisturizing body veil, clarifying bright lotion, snow white body cream, cleansing scrub, silky BHA lotion, sunshine body cream, skin moisturizing gel, liquid pearl bath soap, bath dusting powder, bath and shower gel, highlighting hair gel, conditioner, low pH shampoo, sparkle hair spray, festival hair gel, iridescent hair conditioner, conditioning shampoo, pearly foundation cream, blush stick, face bronze pressed powder, silky finish loose face powder, liquid makeup, body veil, earthtone makeup powder, liquid foundation, creamy peral blush, sheer leg makeup, face bronzer with sunscreen, silky face powder, shimmering pearl pressed powder blush, all purpose color stick, dual face powder, radiance pressed powder blush, gel blush frost, sparkling ruby nail enamel, poured eye shadow, cream eye shadow, emulsion cream mascera, eye shadow, liquid eyeliner, pressed powder eye shadow, emulsion cream eye shadow, charcoal silky powder eyeliner, loose powder eye shadow, concealer, sheer satin pressed powder eye shadows, crayon eye shadow, velvety pressed powder eye shadow, waterproof mascara, slender stick eye shadow, silky pressed powder eye shadow, lipstick, fair taupe lipstick, lip gloss with sunscreen, glossy lipstick, shimmering brick lipstick, lip pomade, soft lipstick, pressed lip powder, long wear lipstick, lipstick with sunscreen, lip glaze-formulation.

The invention is characterized in that 0,01 - 30 weight-% glasses or color glass or glass ceramics or ceramics or glass powders or color glass powders or glass ceramic powders or ceramic powders or composite materials comprising glass or glass ceramics or ceramic powders or mixtures of glass, ceramic and glass ceramic powders.

EP 1 364 639 A2

**EP 1 364 639 A2**

**Description**

The invention relates to cosmetic formulations with glasses or color glass or glass ceramics or ceramics or glass powders or color glass powders or glass ceramic powders or ceramic powders or composite materials comprising glass or glass ceramics or ceramic powders or mixtures of glass, ceramic and glass ceramic powders.

Glasses could be e.g. glasses with antimicrobial, anti inflammatory, light absorbion, light scattering, light reflecting, mineral building, bioactive and coloring properties.

Ceramics could be e.g. oxide ceramics like Alumina, Zirconia, Mica (e.g. inorganic pigments), Silicates, Talc or Non oxide ceramics like Boronnitide, Aluminumnitride and Siliconcarbide.

All powders could be coated with inorganic functional layers e.g. $TiO_2$, $SiO_2$ and silane or organic functional layers.

The powders could be mixed with organic or inorganic pigments for coloring.

Powders include powders, spheres, granulates, nanoparticles, flakes and fibers.

Antimikrobiell glasses are known e.g. from US 5,290,544, US 6,143,318 , JP 10218637, JP 08245240, JP 07291654, JP 03146436, JP 2000264674, JP 2000203876, DE 19932238, der DE 19932239 und der WO 01/0365.

The essential feature of bioactive glass are known for the man skilled in the art and described e.g. in US 5,074,916. Bioactive glasses are characterized in that living tissue is bound by it. Bioactive glass e.g. bounds to the tissue thereby providing for a Hydroxyl-Apatit-Layer.

Color glasses are glasses with a color effect. This color effect can be produced e.g. by ions or nanoparticles. The nanoparticles can be metallic nanoparticles or semiconductor nanoparticles. Color glasses are used for coloring cosmetic products or for absorbing UV- or IR-radiation.

Object of the invention is to provide for cosmetic formulations with glasses or color glass or glass ceramics or ceramics or glass powders or color glass powders or glass ceramic powders or ceramic powders or composite materials comprising glass or glass ceramics or ceramic powders or mixtures of glass, ceramic and glass ceramic powders.

This object is solved by one claim 1.

According to the invention glasses or color glass or glass ceramics or ceramics or glass powders or color glass powders or glass ceramic powders or ceramic powders or composite materials comprising glass or glass ceramics or ceramic powders or mixtures of glass, ceramic and glass ceramic powders can be used in different cosmetic formulations for cosmetic products such as:

transparent pearly lotion, gel cologne with sparkles, pearlescent waterproof sun creme, bronze self tanning cream, sunscreen creme, pearl bronze/copper suntan cream, Icy gel toothpaste, face mask for oily skin, moisturizing body veil, clarifying bright lotion, snow white body cream, cleansing scrub, silky BHA lotion, sunshine body cream, skin moisturizing gel, liquid pearl bath soap, bath dusting powder, bath and shower gel, highlighting hair gel, conditioner, low pH shampoo, sparkle hair spray, festival hair gel, iridescent hair conditioner, conditioning shampoo, pearly foundation cream, blush stick, face bronze pressed powder, silky finish loose face powder, liquid makeup, body veil, earthtone makeup powder, liquid foundation, creamy peral blush, sheer leg makeup, face bronzer with sunscreen, silky face powder, shimmering pearl pressed powder blush, all purpose color stick, dual face powder, radiance pressed powder blush, gel blush frost, sparkling ruby nail enamel, poured eye shadow, cream eye shadow, emulsion cream mascera, eye shadow, liquid eyeliner, pressed powder eye shadow, emulsion cream eye shadow, charcoal silky powder eyeliner, loose powder eye shadow, concealer, sheer satin pressed powder eye shadows, crayon eye shadow, velvety pressed powder eye shadow, waterproof masara, slender stick eye shadow, silky pressed powder eye shadow, lipstick, fair taupe lipstick, lip gloss with sunscreen, glossy lipstick, shimmering brick lipstick, lip pomade, soft lipstick, pressed lip powder, long wear lipstick, lipstick with sunscreen, lip glaze.

Examples of basic cosmetic formulations for the above mentioned cosmetic products from Engelhard Corporation, 101 Wood Avenue, P.O.-Box 707 Iselin, NJ 08830-0770 are given in the appendix to the application.

The basic cosmetic formulations can be applied in colour cosmetics, hair, skin, and nail. The individual components of the basic cosmetic formulation are numerous and varied, but are also well known to one skilled in the art.

The basic cosmetic composition may comprise one or more of the members selected from the group consisting of acidifying agents, alkalizing agents, aerosol propellants, antimicrobial agents, antioxidants, buffering agents, chelating agents, coloring additives, dermotologicaly active agents, dispersing agents, emollients, emulsifying agents, humectants, fragrances, preservatives, sugars, sunscreen agents, surfactants, suspending agents, thickening agents, an vehicles. These ingredients are discussed below. Examples of these agents are listed below as well as in the International Cosmetic Ingredient Dictionary and Handbook, eds. Wenninger and McEwen (The Cosmetic, Toiletry, and Fragrance Assoc., Washington, D.C., 7.sup.th Edition, 1997) (hereinafter "ICT Handbook").

Acidifying and alkalizing agents are added to obtain the desired pH of the composition. Examples of is acid-

2

ifying agents included citric acid, lactic acid, glycolic acid, acetic acid, glacial acetic acid, malic acid, and proprionic acid. Examples of alkalizing agent include edetol, potassium carbonate, potassium hydroxide, sodium borate, sodium carbonate, sodium citrate, sodium lactate, sodium glycolate, and sodium hydroxide. Other acidifying and alkalizing agents are listed on page 1653 of the ICT handbook.

Aerosol propellants are used when the composition is to be administered as an aerosol under pressure. Examples of aerosol propellants include halogenated hydrocarbons such as dichlorodifluoromethane, dichlorotetrafluoroethane, and trichloromonfluoromethane, nitrogen, and volatile hydrocarbons such as butane, propane, isobutane, or mixtures thereof. Other aerosol propellants are listed on page 1655 of the ICT handbook.

Antimicrobial agents are used when the area that the composition is to be applied is prone to microbial infection, e.g., by bacteria, fungal, or protozoa. Examples of such agents include benzyl alcohol, chlorobutanol, phenylethyl alcohol, phenylmercuric acetate, potassium sorbate, and sorbic acid, benzoic acid, butylparaben, ethylparaben, methylparaben, propyl paraben, and sodium benzoate. Other antimicrobial agents are listed on page 1612 of the ICT handbook.

Antioxidants are used to protect ingredients of the composition from oxidizing agents that are included within or come in contact with the composition. Examples of antioxidants include water soluble antioxidants such as grape seed extract, camellia oleifera extract, N-acetyl-L-cysteine, ascorbic acid, sodium sulfite, sodium formaldehyde, isoascorbic acid, cysteine hydrochloride, 1,4-diazobicyclo-(2,2,2)-octane, and mixtures thereof. Examples of oil-soluble antioxidants include ascorbyl palmitate, butytlated hydroxyanisole, butylated hydroxytoluene, potassium propyl gallate, octyl gallate, dodecyl gallate, phenyl-.alpha.-napthyl-amine, and tocopherols such as .alpha.-tocopherol. Other antioxidants are listed on pages 1612-13 of the ICT Handbook.

Buffering agents are used to maintain an established pH of the composition. Examples of buffering agents included calcium acetate, potassium metaphosphate, potassium phosphate monobasic, sodium citrate, and tataric acid. Other buffering agents are listed on page 1612 of the ICT handbook.

Chelating agents are used to maintain the ionic strength of the composition and/or bind to destructive compounds and metals that are included within or come in contact with the composition. Examples of chelating agents included edatate dipotassium, edetate disodium, edetic acid, and ethylenediamine tetracetic acid (EDTA) and its salts (e.g., tetrasodium EDTA). Other chelating agents are listed on page 1626 of the ICT handbook.

Coloring additives are used to add color to the composition in order to help the user identify the area in which the composition has been applied and/or modify the tanning color produced by the self-tanning agent in the composition. Examples of such coloring additives include caramel, carmine, fluorescein derivatives, methoxsalen, trioxsalen, carbon black, azo dyes, anthraquinone dyes, blue azulenes, guajazulene, chamuzulene, erythrosin, bengal rose, phloxin, cyanosin, daphinin, eosin G, cosin 10B, and Acid Red 51. Other coloring additives are listed on page 1628-30 of the ICT handbook. As discussed above, it is preferred not to use coloring additives which contain nitrogen or metals.

Dermatologically active agents include agents for treating wound healing, inflammation, acne, psoriasis, cutaneous aging, skin cancer, impetigo, herpes, chickenpox, dermatitis, pain, itching, and skin irritation. Examples of such dermatologically active agents include hydrocortisone, dexamethesone, panthenol, phenol, tetracycline hydrochloride, yeast, hexylresorcinol, lamin, kinetin, betamethasone, triamcinolone, fluocinolone, methylprednisolone, retinoids such as retinol and retinoic acid, dapsone, sulfasalazine, resorcinol, salicylic acid, benzoyl peroxide, erythromycin-benzoyl peroxide, erythromycin, clindamycin, mupirocin, griseofulvin, azoles such as miconazole, econazole, itraconazole, fluconazole, and ketoconazole, ciclopirox, allylamines such as naftifine and terfinafine, acyclovir, famciclovir, valacyclovir, benzocaine, lidocaine, dibucaine, pramoxine hydrochloride, methyl salicylate, camphor, menthol, resocinol, and vitamins such as tocopherol, tocopheryl acetate, pantothenic acid, panthenol, ascorbic acid, biotin, and retinoids such as retinol, retinoic acid, retinal, retinyl acetate, and retinyl palmitate, alpha.-hydroxy acid, a .beta.-hydroxy acid, or poly-hydroxy acid such as glycolic acid, lactic acid, citric acid, malic acid, and azaleic acid

Examples of dispersing and suspending agents include poligeenan, magnesium aluminum silicate, xanthum gum, and silicon dioxide. Other dispersing or suspending agents are listed on page 1612 of the ICT handbook.

Emollients are agents which soften and smooth the skin. Examples of emollients include hydrocarbon oils and waxes such as mineral oil, petrolatum, microcrystaline wax, polyethylene, triglyceride esters such as those of castor oil, cocoa butter, safflower oil, cottonseed oil, corn oil, olive oil, cod liver oil, almond oil, avocado oil, palm oil, sesame oil, squalene, and soybean oil, acetylated monoglycerides, ethoxylated glycerides, fatty acids, alkyl esters of fatty acids, alkyl esters of benzoic acid (e.g., benzoates), alkenyl esters of fatty acids, fatty alcohols, fatty alcohol ethers, ether-esters, lanolin and derivatives of lanolin, polyhydric alcohol esters, wax esters such as beeswax, vegetable waxes, phospholidds, and sterols. Other emollients are listed on pages 1656-61 of the ICT handbook.

Emulsifying agents are used for preparing the oil-in-water emulsions of the present invention. Examples of emulsifying agents include Arlacel sub. 165. TM. and methyl gluceth sesquisterate, fatty alcohols, fatty alcohols and alkyl phenols condensed with ethylene oxide. Other emulsifiers are listed on pages 1679-87 of the ICT Handbook. Emulsion stabilizers are listed on pages 1634-35 of the ICT Handbook.

Humectants are agents which promote the retention of moisture, e.g., moisturizers. Examples of humectants include sorbitol, glycerin, glycereth 5 lactate, glycereth 7 triacetate, glycereth 7 diisononoate, hexanetriol, glycols such as methyl-propanediol, 1,2-pentanediol, hexylene glycol, and propylene glycol, alkoxylated glucose, D-panthenol and derivatives thereof, and hyaluronic acid. Other humectants are listed on pages 1661-62 of the ICT Handbook.

Examples of fragrances include peppermint, rose oil, rose water, aloe vera, clove oil, menthol, camphor, eucalyptus oil, and other plant extracts. Certain fragrances may require a solubilizer, e.g., PPG-5-ceteth-20. To eliminate certain odors from compositions, masking agents may be used. An example of a masking agent includes ethylene brassylate. Other fragrances and masking agents are listed on pages 1639-40 of the ICT Handbook.

Preservatives are used to protect the composition from degradation. Examples of preservatives include phenoxyethanol, benzoic aicd, benzyl alcohol, parabens such as methylparaben, propylparaben, butylparaben, isopropylparaben, and isobutylparaben, diazolidinyl urea, imidazolidinyl urea, diazolindyl urea, benzalkonium chloride, benzethonium chloride, phenol, and mixtures thereof (e.g., the paraben mixture Liquipar Oil.TM.). Other preservatives are listed on pages 1654-55 of the ICT Handbook.

Sugars are used to improve the results obatined by the self-tanning agents. Examples of sugars include monosaccharides, disaccharides, sorbitol, and polysccharides such as glucose, xylose, fructose, reose, ribose, pentose, arabinose, allose, tallose, altrose, mannose, galactose, lactose, sucrose, erythrose, glyceraldehyde, or any combination thereof.

Sunscreen agents are agents used to screen or reduce the amount of ultraviolet radiation impinging on the skin (e.g., by absorption, scattering, and reflection of the ultraviolet radiation). Segarin, et al., Cosmetics Science and Technology, Chapter VIII, pages 189, et seq. discloses numerous examples of sunscreen agents. Examples of sunscreen agents include both organic compounds and their salts such as phenylbenzimidazole sulfonic aicd, octyl methoxycinnamate, octyl salicylate, benzophenones such as benzophenone-3, homosalate, octocrylene, avobenzone, and menthyl anthranilate, as well as inorganic particulate materials such as zinc oxide and titanium dioxide. Other sunscreen agents are listed on page 1672 of the ICT Handbook. Generally, the composition will contain from about 1 % to about 50%, by weight, of sunscreen agent(s). The exact amounts will vary depending on the sunscreen used and the desired sun-protection factor (SPF), e.g., and SPF of at least 4 or an SPF of at least 15.

Surfactants are agents used to stabilize multi-component compositions, e.g., used as wetting agents, antifoam agents, emulsifiers, dispersing agents, and penetrants. Examples of surfactants include alkene oxide, ethers of fatty alcohols, glucose, and sorbitol, methyl gluceth 20, decyl polyglucoside, laureth 4, laureth 9, monoethanolamine, nonoxynol 4, nonoxynol 9, nonoxynol 10, nonoxynol 15, nonoxynol 30, poloxalene, polyoxyl 8, 40, and 50 stearate, polysorbate 20, polysorbate 40, polysorbate 60, polysorbate 65, polysorbate 80, and polysorbate 85, sodium lauryl sulfate, sorbitan and its derivatives. Other surfactants are listed on page 1672-90 of the ICT Handbook.

Vehicles are often referred to as the base for the basic cosmetic composition, e.g., a fluid that is capable of delivering the other components of the composition to the skin with acceptable absorption of those components into the skin. Examples of vehicles include water (e.g., deionized water), oil-in-water emulsions (e.g., where the continuous water phase contains the water soluble agents and the discontinuous oil phase contains the oil soluble agents), and water-in-oil emulsions (e.g., where the continuous oil phase contains the oil soluble agents and the discontinuous water phase contains the water soluble agents). The oil phase may be established by the addition of an animal/vegetable derived oil, ester, or ether, a hydrocarbon and/or silicone solvents, e.g., dimethicone and cyclomethicone, together with various emulsifying agents.

The basic cosmetic formulation may be in a number of different delivery forms, e.g., a spray, mist, aerosol, semi-solid cream, liquid such as a solution, emulsion, or suspension, lotion, gel, solid such as a powder, adherent stick, flexible mask, or self-hardening liquid or gel, or other suitable forms intended to be-applied to the hair, skin, or nails of a subject. Water-in-oil emulsions (e.g., ratio of about 10:1 to about 1:100 such as about 1:1 to about 1:10) and oil-in-water emulsions (e.g., ratio of about 10:1 to about 1:100 such as about 1:1 to about 1:10) are typically used in preparing lotions and creams.

The viscosity of the basic cosmetic composition depends upon the type of formulation being prepared, e.g., a liquid formulation will have a higher viscosity than a cream or gel formulation. Typically, the viscosity of cream formulations of the present invention will range from 5,000 to 150,000 cps (e.g., about 10,000 to about 40,000 cps). Bulking agents may be used to increase the viscosity of the composition. An example of a bulking agent is talc, magnesium aluminum salicate, and starches Other bulking agents are listed on page 1625-26 of the ICT Handbook. Other viscosity increasing agents are listed on pages 1693-97 of the ICT Handbook. Viscosity decreasing agents are listed on pages 1692-92 of the ICT Handbook.

The basic cosmetic formulation may be prepared using methodology that is well known by an artisan of ordinary skill (e.g., by using well-known mixing and blending procedures). For examples, for emulsion products of the present invention, each phase of the emulsion may be separately prepared with all of the components contained in their appropriate phases. The emulsion is then formed by adding one phase to the other phase with agitation.

The composition or product of the present invention may be packaged in a container that is well known by an artisan of ordinary skill, e.g., in a polyethylene or PVC tube with a dispensing cap.

The composition of the present invention may be prepared using methodology that is well known by an artisan of ordinary skill.

The basis glass for the glasses or color glass or glass ceramics or glass powders or color glass powders or glass ceramic powders or composite material which are mixed with the above mentioned cosmetic formulations comprise advantageously the following components.

$SiO_2$ 0 - 80 weight-%
$Na_2O$ 0 - 35 weight-%
$P_2O_5$ 0 - 80 weight-%
MgO 0 - 30 weight-%
$Ag_2O$ 0 - 2 weight-%
AgJ 0 - 0,5 weight-%
NaJ 0 - 5 weight-%
$TiO_2$ 0 - 5 weight-%
$K_2O$ 0 - 35 weight-%
ZnO 0 - 20 weight-%
$Al_2O_3$ 0 - 25 weight-%
$B_2O_3$ 0 - 25 weight-%

Furthermore glasses with components given above can comprise ions as Fe, Co, Cr, V, Ce, Cu, Mn, Ni, Bi, Sn, Ag, Au, J up to a sum of 10 Gew-%. By this components a color effect or a anti-inflammatory effect can be achieved or a antimicrobiel glass can be obtained.

A color glass can comprise the following components:

$SiO_2$ 0 - 80 weight-%
$Na_2O$ 0 - 35 weight-%
$P_2O_5$ 0 - 80 weight-%
MgO 0 - 5 weight-%
$Ag_2O$ 0 - 0,5 weight-%
AgJ 0 - 0,5 weight-%
NaJ 0 - 5 weight-%
$TiO_2$ 0 - 5 weight-%
$K_2O$ 0 - 35 weight-%
ZnO 0 - 10 weight-%
$Al_2O_3$ 0 - 25 weight-%
$B_2O_3$ 0 - 25 weight-%
SnO 0 - 5 weight-%
$CeO_2$ 0 - 3 weight-%
Au 0,001-0,1 weight-%

The glasses can also be used in composite materials comprising nanoparticles such as $TiO_2$, ZnO, $ZrO_2$, metallic gold or silver. The nanoparticles are smaller than 500nm, smaller than 200 nm, smaller than 100 nm, smaller than 50 nm, preferably smaller than 10 nm in size. The composit materials comprise nanoparticles up to 30 weight-%, up to 15 weight-%, up to 5 weight-% preferably up to 1 weight-% and most preferably up to 0,1 weight-%.

Preferred embodiments of the above mentioned glasses are given in table 1.

The glasses can be pulverized to glass powder. The particle size of the glass powder is < 100 μm, preferably < 50 μm 20 μm, most preferable < 10 μm. Advantagously the glass particles are < 1 μm. Glass powder of different siza can be combined.

Instead of glasses glass-ceramic-materials can be used. Glasses as given above and especially in table 1 are ceramized under temperatures 50- 400°C above $T_g$. The amount of the crystalline phase in the glass ceramic material is preferably greater than 5 weight-%, preferably greater than 30 weight-%, most preferably greater than 50 weight-%.

Usually the glass ceramic material is used as glass ceramic powder.

Glass ceramic powder can be obtained either by pulverizing the glass first and then ceramizing the pulverized glass powder or first ceramizing a block of glass and thereafter pulverizing the block or ribbon of glass ceramic material.

The particle size of the ceramic powder is < 500μm, preferably < 100μm, more preferably < 50 μm , most preferable < 10 μm. Advantagously the glass particles are < 1 μm. Glass powder of different size can be combined.

The invention will be described now by special embodiments without being restricted to them.

The preferred glasses as a basis for the glasses or color glass or glass ceramics or ceramics or glass powders or color glass powders or glass ceramic powders or ceramic powders or composite materials comprising glass or glass ceramics or ceramic powders or mixtures of glass, ceramic and glass ceramic powders which are mixed with basic cosmetic formulations as given in the appendix are shown in table 1.

In the appendix examples for basic cosmetic formulations for transparent pearly lotion, gel cologne with sparkles, pearlescent waterproof sun creme, bronze self tanning cream, sunscreen creme, pearl bronze/copper suntan cream, Icy gel toothpaste, face mask for oily skin, moisturizing body veil, clarifying bright lotion, snow white body cream, cleansing scrub, silky BHA lotion, sunshine body cream, skin moisturizing gel, liquid pearl bath soap, bath dusting powder, bath and shower gel, highlighting hair gel, conditioner, low pH shampoo, sparkle hair spray, festival hair gel, iridescent hair conditioner, conditioning shampoo, pearly foundation cream, blush stick, face bronze pressed powder, silky finish loose face powder, liquid makeup, body veil, earthtone makeup powder, liquid foundation, creamy peral blush, sheer leg makeup, face bronzer with sunscreen, silky face powder, shimmering pearl pressed powder blush, all purpose color stick, dual face powder, radiance pressed powder blush, gel blush frost, sparkling ruby nail enamel, poured eye shadow, cream eye shadow, emulsion cream mascera, eye shadow, liquid eyeliner, pressed powder eye shadow, emulsion cream eye shadow, charcoal silky powder eyeliner, loose powder eye shadow, concealer, sheer satin pressed powder eye shadows, crayon eye shadow, velvety pressed powder eye shadow, waterproof masara, slender stick eye shadow, silky pressed powder eye shadow, lipstick, fair taupe lipstick, lip gloss with sunscreen, glossy lipstick, shimmering brick lipstick, lip pomade, soft lipstick, pressed lip powder, long wear lipstick, lipstick with sunscreen, lip glaze-formulations are given which can be mixed with the glasses or color glass or glass ceramics or ceramics or glass powders or color glass powders or glass ceramic powders or ceramic powders or composite materials comprising glass or glass ceramics or ceramic powders or mixtures of glass, ceramic and glass ceramic powders are given.

In a preferred embodiment 99,9 weigth-%- 70 weight-% of a basic cosmetic formulation as given for example in the appendix is mixed with about 0,01 - 30 weight-% bioactive glasses, color glass, glass-powders, color glass powders, glassceramic-powders or composit materials comprising glass and nanoparticles.

**Silky Face Powder**

CLF-910959M

| PHASE | INGREDIENTS | %WT. |
|---|---|---|
| A. | Talc | (q.s. to 100%) 55.10 |
| | Mearlmica® SVA (Mica (and) Lauroyl Lysine)[1] | 15.00 |
| | Boron Nitride | 10.00 |
| | Titanium Dioxide (C47-056 Cosmetic White)[2] | 8.50 |
| | Silica (Spherica P-1500)[3] | 2.30 |
| | Iron Oxides (C33-1700 Cosmetic Yellow)[2] | 1.50 |
| | Iron Oxides (C33-225 Cosmetic Brown)[2] | 1.00 |
| | Iron Oxides (C33-1099 Cosmetic Russet)[2] | 0.60 |
| | Preservatives | q.s. |
| B. | Fragrance | q.s. |
| | Antioxidant | q.s. |
| | Isopropyl Palmitate (and) Lanolin Oil (Isopropylan 33)[4]. | 4.50 |
| | Mineral Oil | 1.50 |
| | | 100.00 |

**PROCEDURE**

I. Thoroughly blend and disperse Phase A in appropriate dry blending/dispersing equipment.
II. Spray premixed Phase B into premixed Phase A and continue blending.
III. Pulverize and press.

**Silky Face Powder**

CLF-91 0959M

| TYPICAL PROPERTIES | |
| --- | --- |
| COLOR | Beige |
| ODOR | Characteristic |
| APPEARANCE | Matte |
| TEXTURE | Smooth |
| HARDNESS | 41 ± 5 |
| DROP TEST | Good |
| COVERAGE | Excellent |
| SKIN ADHESION | Very good |

SUPPLIERS

1. **Engenlhard Corporation**
2. Sun Chemical Corporation
3. Ikeda Corporation
4. Amerchol Corporation

**Shimmering Pearl Pressed Powder Blush**

CLF-910155M

| TYPICAL PROPERTIES | |
| --- | --- |
| COLOR | Red |
| ODOR | Characteristic |
| APPEARANCE | Peariescent |
| TEXTURE | Smooth |
| HARDNESS | 46 ± 5 |
| DROP TEST | Excellent |
| COVERAGE | Excellent |
| SKIN ADHESION | Very good |

SUPPLIERS

1. **Engelhard Corporation**
2. Sun Chemical Corporation
3, Witco Corporation
4. Henkel Corporation

**Shimmering Pearl Pressed Powder Blush**

CLF-910155M

| PHASE | INGREDIENTS | | %WT. |
|-------|-------------|---|------|
| A. | Talc | (q.s. to 100%) | 25.20 |
| | Zinc Stearate | | 11.00 |
| | Bi-Lite® 20 BL1070 (Mica (and) Bismuth Oxychloride)[1] | | 11.00 |
| | Pearl-Glo® UVR PG1086 (Bismuth Oxychloride)[7] | | 5.00 |
| | Cloisonné® Cerise Flambe 550Z (Mica (and) Iron Oxides)[1] | | 32.00 |
| | Red 7 Lake (C19-011 Rubine Lake)[2] | | 0.80 |
| | Preservatives | | q.s. |
| B. | Fragrance | | q.s. |
| | Antioxidant | | q.s. |
| | Mineral Oil (Ervol White Mineral Oil)[3] | | 2.50 |
| | Sorbitan Diisostearate (Emsorb 2518)[4] | | 2.50 |
| C. | Timica® Golden Bronze 240A (Mica (and) Iron Oxides' (and) Titanium Dioxide)[1] | | 10.00 |
| | | | 100.00 |

## PROCEDURE

I. Thoroughly blend and disperse Phase A in appropriate dry blending/dispersing equipment.

II. Add Phase B ingredients into a support vessel. Heat and mix until uniform.

III. Spray Phase B into premixed Phase A and continue blending.

IV. Pulverize and return to blender.

V. Add Phase C to Phase A-B and mix until uniform.

**All Purpose Color Stick**

CLL-980177

## PROCEDURE, *CONTINUED*

III. Add in Phase C and mix with constant stirring.

IV. Pour at 75° ± 5°C.

V. Mold, cool and flame the lipsticks.

Note: If iron oxide or organic pigments are used, they should first be dispersed in Castor oil; this mixture should then be milled in either a colloid or roller mill.

| TYPICAL PROPERTIES | |
| --- | --- |
| COLOR | Russet |
| ODOR | Characteristic |
| APPEARANCE | Pearlescent |
| DROOP POINT (44°C) | Passes |
| CAPILLARY POINT | 70° ± 3°C |

SUPPLIERS

1. Scher Chemicals, inc.

2. Stepan Company

3. Lipo Chemicals, Inc.

4. Amerchol Corporation

5. Ikeda Corporation

6. ISP

7. Roche Vitamins and Fine Chemicals

8. **Engdhard Corporation**

**All Purpose Color Stick**

CLL-980177

| PHASE | INGREDIENTS. | | %WT. |
| --- | --- | --- | --- |
| A. | Diisopro Dimer Dilinoleate (Schercemol DID)[1] | | 5.55 |
| | Capryiic/Capric Triglyceride (Neobee M-5)[2] | (q.s. to 100%) | 15.00 |
| | Hydrogenated Vegetable Oil (Lipo 55)[3] | | 14.05 |
| | Isopropyl Lanolate (Amerlate P)[4] | | 15.00 |
| | PEG-4 Diheptanoate (Uponate 2-DH)[3] | | 5.00 |
| | Candelilla Wax | | 5.30 |
| | Carnauba Wax | | 5.30 |
| | Paraffin Wax | | 1.30 |
| | Ozokerite Wax | | 5.30 |
| | Nylon-6 (Orgasol 1002 D N Cos 20 microns)[3] | | 1.00 |
| | Silica (Spherica P-1500)[5] | | 1.00 |
| | Benzophenone-3 (Escalol 567)[6] | | 1.00 |
| | Octyl Methoxycinnamate (Parsol MCX)[7] | | 3.00 |
| | PVP/Hexadecene Copolymer (Ganex V-216)[6] | | 10.00 |
| | Phenoxyethanol (and) Isopropylparaben (and) | | |
| | Isobutylparaben (and) n-Butylparaben (Liquapar PE)[6] | | 1.00 |
| | BHT | | 0.10 |

(continued)

| PHASE | INGREDIENTS. | %WT. |
|---|---|---|
| | Retinyl Palmitate | 0.05 |
| | Tocopheryl Acetate | 0.05 |
| B. | Cloisonné® Sparkle Rouge 450J (Mica (and) Iron Oxides)[8] | 10.00 |
| | Mearlmica® SVA (Mica (and) Lauroyl Lysine)[8] | 1.00 |
| C. | Fragrance | q.s. 100.00 |

**PROCEDURE**

I. Weigh all the ingredients in Phase A into a heated vessel and raise temperature to 85° ± 3°C, stirring until melted and uniform.

II. Add in Phase B and mix until all the pearl pigment is well dispersed.

*(Procedure continued on back.)*

**Dual Face Powder**

CU-910189E

| TYPICAL PROPERTIES | |
|---|---|
| COLOR | Beige |
| ODOR | Characteristic |
| APPEARANCE | Matte |
| TEXTURE | Creamy |
| HARDNESS | 35 ± 5 |
| DROP TEST | Excellent |
| COVERAGE | Very good |
| SKIN ADHESION | Very good |

SUPPLIERS

1. **Engelhard Corporation**
2. Whittaker, dark & Daniels, Inc.
3. Sun Chemical Corporation
4. Hispano Quimica S.A./Centerchem, Inc.
5. Dow Corning Corporation

**Dual Face Powder**

CLF-910189E

| PHASE | INGREDIENTS | %WT. |
|---|---|---|
| A. | Mearitalc® TCA (Talc (and) Lauroyl Lysine)[1] | 70,50 |
| | Mearimica® SVA (Mica (and) Lauroyl Lysine)[1] | 19.50 |
| | Zinc Oxide (Zinc Oxide USP)[2] | 2.00 |
| | Iron Oxides (C33-1700 Cosmetic Yellow)[3] | 0.70 |
| | Iron Oxides (C33-225 Cosmetic Brown)[3] | 1.00 |
| | Iron Oxides (C33-1099 Cosmetic Russet)[3] | 0.30 |
| | Preservatives | q.s. |
| B. | Fragrance | q.s. |
| | Antioxidant | q.s. |
| | Squaiane (Fitoderm)[4] | 2.00 |
| | Dimethicone (Dow Corning 200 Fluid)[5] | 0.50 |
| | Mineral Oil | 3.50 |
| | | 100.00 |

PROCEDURE

I. Thoroughly blend and disperse Phase A in appropriate dry blending/dispersing equipment.

II. Spray Phase B into premixed Phase A and continue blending.

III. Pulverize and press.

**Radiance Pressed Powder Blush**

CLF-930163

| TYPICAL PROPERTIES | |
|---|---|
| COLOR | Pink |
| ODOR | Characteristic |
| APPEARANCE | Matte |
| TEXTURE | Creamy |
| HARDNESS | 25 ± 5 |
| DROP TEST | Excellent |
| COVERAGE | Excellent |
| SKIN ADHESION | Very good |

SUPPLIERS

    1. **Engelhard Corporation**
    2. Sun Chemical Corporation
    3. Witco Corporation
    4. Stepan Company

**Radiance Pressed Powder Blush**

CLF-930163

| PHASE | INGREDIENTS | | %WT. |
|-------|-------------|---|------|
| A. | Mearltalc® TCA (Talc (and) Lauroyl Lysine)[1] | (q.s. to 100%) | 53.40 |
| | Mearimica® SVA (Mica (and) Lauroyi Lysine)[1] | | 10.00 |
| | Boron Nitride | | 5.00 |
| | Zinc Stearate | | 3.00 |
| | Magnesium Carbonate | | 3.00 |
| | Red 6 Lake (C19-022 Light Rubine Lake)[2] | | 0.80 |
| | Red 7 Lake (C19-011 Rubine Lake)[2] | | 0.30 |
| | Cloisonné® Red 424C (Mica (and) Titanium Dioxide (and) Carmine)[1] | | 6.00 |
| | Duocrome® RB (Red/Blue) 624C (Mica (and) Titanium Dioxide (and) Carmine)[1] | | 7.00 |
| | Flamenco® Gold 220C (Mica (and) Titanium Dioxide)[4] | | 0.50 |
| | Preservatives | | q.s. |
| B. | Fragrance. | | q.s. |
| | Antioxidant | | q.s. |
| | Mineral Oil (Ervol White Mineral Oil)[3] | | 3,00 |
| | Caprylic/Capric Triglyceride (Neobee M-5)[4] | | 2.00 |
| C. | Duocrome RB (Red/Blue) 624C (Mica (and) Titanium Dioxide (and) Carmine)[1] | | 6.00 |
| | | | 100.00 |

PROCEDURE

I. Thoroughly blend and disperse Phase A in appropriate dry Mending/dispersing equipment.

II. Add Phase B ingredients into a support vessel. Heat and mix until uniform.

III. Spray Phase B into premixed Phase A and continue blending.

IV. Pulverize and return to blender.

V. Add Phase C to Phase A-B and mix until uniform.

**Gel-Blush Frost**

CLF-920023

| TYPICAL PROPERTIES | |
| --- | --- |
| COLOR | Ruby |
| ODOR | Characteristic |
| APPEARANCE | Shimmering |
| TEXTURE | Creamy |
| VISCOSITY | >2,000,000 ± 200,000 cps |
| pH | 6.3 ± 0.5 |
| SPECIFIC GRAVITY | 1.13 ± 0.1 |

SUPPLIERS

1. Honeywell International, Inc.
2. Amerchol Corporation
3. Witco Corporation
4. Dow Corning Corporation
5. Frank B. Ross Co., Inc.
6. Lanaetex Products, Inc.
7. **Engdhard Corporation**

**Gel-Blush Frost**

CLF-920023

| PHASE | INGREDIENTS | %WT. |
| --- | --- | --- |
| A. | Ethylene/Vinyl Acetate Copolymer (A-C Copolymer 400)[1] | 9.50 |
| | Acetylated Lanolin Alcohols (Acetulan)[2] | 10.00 |
| | Mineral Oil (Ervol White Mineral Oil)[3] | 10.60 |
| | Lanolin Oil (Lanogene)[2] | 2.50 |
| | Cyclomethicone (Silicone Fluid 344)[4] | 5.00 |
| | Cyclomethicone (Silicone Fluid 345)[4] | 2.50 |
| | Isopropyl Lanolate (Amerlate P)[2] | 4.50 |
| | Ozokerite (Ozokerite Wax 170 D)[5] | 2.00 |
| | Beeswax (Beeswax Synthethic Cosmetic Grace)[5] | 2.00 |
| | Isopropyl Myristate | 15.10 |
| | Lanolin Alcohol (Super Anatol)[6] | 8.00 |
| | Antioxidant | q.s. |
| B. | Gemtone® Tan Opal G005 (Mica (and) Iron Oxides (and) Titanium Dioxide)[7] | 5.30 |
| | Gemtone Ruby G010 (Mica (and) Titanium Dioxide (and) Carmine)[7] | 10.00 |
| | Gemtone Garnet G009 (Mica (and) Titanium Dioxide | |

(continued)

| PHASE | INGREDIENTS | %WT. |
|---|---|---|
| | (and) Iron Oxides (and) Carmine)[7] | 15.00 |
| | Preservatives | q.s. |
| C. | Fragrance | q.s. |
| | | 100.00 |

**PROCEDURE**

I. Heat combined Phase A to 83° ± 3°C with stirring until a clear solution is obtained.

II. Add Phase B to Phase A and stir until uniform.

III. Cool to 62° ± 3°C and add Phase C.

IV. Continue mixing and fill at 50° ± 5°C.

**Sparkling Ruby Nail Enamel**

CLN-922853M

PROCEDURE

I. Combine Phase A ingredients in a vessel fitted with a propeller type mixer and stir until uniform.
II. Combine Phase B ingredients (thinners) in a secondary vessel and mix well until uniform.
III. Add Phase B to Phase A while stirring until uniform.

| TYPICAL PROPERTIES | |
|---|---|
| COLOR | Dark red with frosted highlights |
| ODOR | Characteristic solvent odor |
| IDENTITY | Nitrocellulose in solvent mix |
| VISCOSITY | 180 ± 10 cps at 25°C |
| SPECIFIC GRAVITY | 0.95 ± 0.05 at 25°C |

SUPPLIERS

1. **Engelhard Corporation**

2. Dow Corning Corporation

5. Telechemische, Inc.

**Sparkling Ruby Nail Enamel**

CLN-922853M

| PHASE | INGREDIENTS | %WT. |
|---|---|---|
| A. | Suspending Lacquer SLF-2 (Butyl Acetate (and) Toluene (and) Nitracellulose (and) Tosylamide/Formaldehyde Resin (and) Isopropyl Alcohol (and) Dibutyl Phthalate (and) Ethyl Acetate (and) Camphor (and) n-Butyl Alcohol (and) Silica (and) Quaterinum-18 Hectorite)[1] | 80.00 |
| | Biju® Ultra UXD (Butyl Acetate (and) Bismuth Oxychloride (and) Nitrocellulose (and) Toluene (and) Isopropyl Alcohol (and) Steara Konium Hectorite)[1] | 2.00 |
| | Flamenco® Ultra Sparkle 4500 (Mica (and) Titanium Dioxide)[1] | 1.00 |
| | Red 6 Lake (7.9% Dispersion in Nitrocellulose Base, CB-70) | 6.00 |
| | Red 7 Lake (7.9% Dispersion in Nitrocellulose Base, CB-11) | 2.00 |
| | Red 34 Lake (7.9% Dispersion in Nitrocellulose Base, CB-91)[2] | 0.75 |
| | Dimethicone (Dow Corning 200 50cs. - 1% Butyl Acetate)[2] | 1.00 |
| | Tosylamide/Epoxy Resin (Lustrabrite S-70)[3] | 4.00 |
| B. | Thinners* | 3.25 |
| | | 100.00 |
| | * Butyl Acetate (Urethane Grade) | 36.00 |
| | Ethyl Acetate (Urethane Grade) | 13.00 |
| | Toluene | 51.00 |
| | | 100.00 |
| Note: The quantity of Phase B may be varied to compensate for evaporation or viscosity adjustment. | | |

**Poured Eye Shadow**

CLE-911016M

| TYPICAL PROPERTIES | |
|---|---|
| COLOR | Green |
| ODOR | Characteristic |
| APPEARANCE | Peariy |
| CAPILLARY POINT | 67° ± 3°C |

SUPPLIERS

1. Witco Corporation
2. Dow Corning Corporation
3. **Engelhard Corporation**
4. Ikeda Corporation
5. Sun Chemical Corporation

**Poured Eye Shadow**

CLE-911016M

| PHASE | INGREDIENTS | | %WT. |
|---|---|---|---|
| A. | Isopropyl Myristate | (q.s. to 100%) | 20.40 |
| | Mineral Oil (Carnation White Mineral Oil)[1] | | 10.00 |
| | Lauryl Lactate | | 10.00 |
| | Ozokerite Wax | | 8.00 |
| | Carnauba Wax | | 4.00 |
| | Dimethicone (Dow Corning 200 Fluid - 350 cs.)[2] | | 4.00 |
| | Preservatives & Antioxidants | | q.s. |
| B. | Mearlmica® SVA (Mica (and) Lauroyl Lystine)[3] | | 14.60 |
| | Flamenco® Velvet 120V (Mica (and) Titanium Dioxide)[3] | | 2.00 |
| | Cloisonné® Super Green 827C (Mica (and) Titanium Dioxide (and) Iron Oxides (and) Ferric Ferrocyanide)[3] | | 6.00 |
| | Silica (Spherica P-1500)[4] | | 15.00 |
| | Corn Starch | | 2.00 |
| | Ferric Ammonium Ferrocyanide (C38-5410 Cosmetic Blue F)[5] | | 2.70 |
| | Iron Oxides (C33-210 Cosmetic Yellow)[5] | | 1.30 |
| | | | 100.00 |

PROCEDURE

I. Add Phase A ingredients to a heated vessel and stir bringing temperature to $80° \pm 3°C$.

II. Pulverize Phase B ingredients and add to Phase A with thorough agitation.

III. Pour into holding kettles or appropriate compacts.

**Cream Eye Shadow**

CLE-920003

| TYPICAL PROPERTIES | |
|---|---|
| COLOR | Green |
| ODOR | Characteristic |
| APPEARANCE | frosted |
| TEXTURE | Smooth |
| IDENTITY | Oil/water emulsion |
| VISCOSITY | $5,100 \pm 500$ cps |
| pH | $7.30 \pm 0.5$ |
| SPECIFIC GRAVITY | $1.17 \pm 0.10$ |

SUPPLIERS

1. R. T. Vanderbilt Company, inc.
2. **Engelhard Corporation**
3. Aqualon
4. Dow Chemical Company
5. Lonza, Inc.
6. Crode, Inc.

**Cream Eye Shadow**

CLE-920003

| PHASE | INGREDIENTS | | %WT. |
|---|---|---|---|
| A. | Water | (q.s. to 100%) | 63.90 |
| | Magnesium Aluminum Silicate (Veegum)[1] | | 1.50 |
| B. | Cloisonné® Green 828C (Mica (and) Titanium Dioxide (and) Chromium Oxide Greens)[2] | | 12.50 |
| | Cloisonné Nu-Antique Green 828CB (Mica (and) Titanium Dioxide (and) Iron Oxides (and) Oxide Greens)[2] | | 2.00 |
| | Mearimica® SVA (Mica (and) Lauroyl Lysine)[2] | | 5.50 |
| C. | Propylene Glycol | | 8.00 |
| | Cellulose Gum (CMC-7LF)[3] | | 1.00 |
| D. | Triethanolamine (TEA 99%)[4] | | 0.80 |
| | Preservatives (water soluble) | | q.s. |
| E. | Stearic Acid | | 3.50 |
| | Glyceryl Stearate (Aldo MS)[5] | | 0.80 |
| | Cleyl Alcohol (Novol)[6] | | 0.50 |
| | Preservatives (oil soluble) | | q.s. |
| | | | 100.00 |

PROCEDURE

I. Disperse Veegum into water using high-shear mixing until smooth.

II. Blend Phase B and add to Phase A, mixing until thoroughly dispersed.

III. Add Phase C slurry to Phase A-B and mix until smooth.

IV. Add Phase D to Phase A-B-C with gentle agitation and heat to 75° ± 5°C.

V. In a support vessel heat Phase E ingredients to 75° ± 5°C with gentle agitation.

VI. Add Phase E to Phase A-B-C-D with gentle agitation, maintaining temperature at 75° ± 5°C.

VII. Maintain constant agitation and cool batch to 35° ± 5°C; store or fill into appropriate containers.

**Emulsion Cream Mascara**

CLE-920012M

| TYPICAL PROPERTIES | |
|---|---|
| COLOR | Olive |
| ODOR | Characteristic |
| APPEARANCE | Frosted |
| TEXTURE | Smooth - creamy |
| IDENTITY | Oil/water emulsion |
| VISCOSITY | 111,200 ± 11,000 cps |
| pH | 7.80 ± 0.5 |
| SPECIFIC GRAVITY | 1.1 ± 0.10 |

SUPPLIERS

1. Dow Chemical Company
2. **Engelhard Corporation**
3. Sun Chemical Corporation
4. Frank B. Ross Co., Inc.
5. Henkel Corporation

**Emulsion Cream Mascara**

CLE-920012M

| PHASE | INGREDIENTS | | %WT. |
|---|---|---|---|
| A. | Water | (q.s. to 100%) | 47.50 |
| | Triethanolamine (TEA 99%)[1] | | 2.00 |
| | Propylene Glycol | | 15.00 |
| B. | Flamenco® Gold 220C (Mica (and) Titanium Dioxide)[2] | | 10.20 |
| | Iron Oxides (C33-4799 Cosmetic Black)[3] | | 1.80 |
| C. | Candelilla Wax (Refined, Candelilla Wax)[4] | | 12.00 |
| | Stearic Acid (Emersol 132)[5] | | 4.50 |
| | Isostearic Acid (Emersol 871)[5] | | 4.50 |
| | Preservatives (oil soluble) | | q.s. |
| D. | Water | | 2.50 |
| | Preservatives (water soluble) | | q.s. |
| | | | 100.00 |

PROCEDURE

I. Add Phase A ingredients to the main vessel.

II. Add Phase B to Phase A with agitation, and raise the temperature to 75° ± 3°C.

III. Add Phase C to the support vessel and mix while raising temperature to 80° ± 3°C.

IV. When the water and oil phases are both at 75° ± 3°C, add Phase C to Phase A-B and maintain sweep agitation.

V. With constant agitation cool batch to 50°C and add premixed Phase D solution to Phase A-B-C.

VI. Continue stirring and cooling to 40°C; batch may be filled below this temperature.

**"Touch of Velvet" Eye Shadow**

CLE-922277M

| TYPICAL PROPERTIES | |
|---|---|
| COLOR | Purple |
| ODOR | Characteristic |
| APPEARANCE | Matte |
| TEXTURE | Velvety |
| HARDNESS | 42 ± 5 |
| DROP TEST | Excellent |
| COVERAGE | Good |
| SKIN ADHESION | Very good |

SUPPLIERS

1. **Engeldhard Corporation**
2. Sun Chemical Corporation
3. Witco Corporation
4. Lipo Chemicals, Inc.
5. Bernel Chemical Company, inc.

**"Touch of Velvet". Eye Shadow**

CLE-922277M

| PHASE | INGREDIENTS | | %WT. |
|---|---|---|---|
| A. | Mearitalc® TCA (Talc(and) Lauroyl Lysine)' | (q.s. to 100%) | 39.50 |
| | Mearlmica® SVA (Mica (and) Lauroyl Lysine)[1] | | 25,00 |
| | Manganese Violet (C43-001 Mango Violet)[2] | | 15.00 |
| | Calcium Stearate (Calcium Stearate Regular)[3] | | 5.00 |
| | Pearl-Glo® PG1085 (Bismuth Oxychloride)[7] | | 5.00 |
| | Nylon-12 (Orgasol 2002D Ex Nat Cos)[4] | | 2.50 |
| | Ultramarines (C43-1810 Cosmetic Blue)[2] | | 2.00 |
| | Iron Oxides (C33-4799 Cosmetic Black)[2] | | 1.50 |
| | Iron Oxides (C33-8098 Cosmetic Russet)[2] | | 1.50 |
| | Preservatives | | q.s. |
| B. | Cetyl Octanoate (Berne! Ester CO)[5] | | 1.50 |
| | Polydecene | | 1.50 |

(continued)

| PHASE | INGREDIENTS | %WT. |
|---|---|---|
| | Antioxidant | q.s.<br>100.00 |

PROCEDURE

I. Thoroughly blend and disperse Phase A in appropriate dry blending/dispersing equipment.

II. Add Phase B ingredients into a support vessel. Heat and mix until uniform.

III. Spray Phase B into premixed Phase A and continue blending.

IV. Pulverize and mix until uniform.

**Liquid Eyeliner**

CLE-921119

| TYPICAL PROPERTIES | |
|---|---|
| COLOR | Green |
| ODOR | Characteristic |
| APPEARANCE | Frosted |
| TEXTURE | Smooth |
| IDENTITY | Oil/water emulsion |
| VISCOSITY | $416,500 \pm 40,000$ cps |
| pH | $7.30 \pm 0.5$ |
| SPECIFIC GRAVITY | $1.17 \pm 0.1$ |

SUPPLIERS

1. R.T. Vanderbilt Company, Inc.
2. Dow Chemical Company
3 The NutraSweet Kelco Company
4. **Engelhard Corporation**
5. Lonza, Inc.
6. Croda, inc.

**Liquid Eyeliner**

CLE-921119

| PHASE | INGREDIENTS | %WT. | |
|-------|-------------|------|-----|
| A. | Water | (q.s. to 100%) | 65.20 |
| | Magnesium Aluminum Silicate (Veegum)[1] | | 1.00 |
| B. | Triethanolamine (TEA 99%)[2] | | 1.40 |
| | Propylene Glycol | | 8.00 |
| C. | Xanthan Gum (Keitrol T)[3] | | 0.30 |
| | Preservatives (water soluble) | | q.s. |
| D. | Flamenco® Twilight Green 820CB (Mica (and) Titanium Dioxide (and) Iron Oxides)[4] | | 15.00 |
| | Mearlmica® CF (Mica)[4] | | 5.00 |
| E. | Stearic Acid | | 2.80 |
| | Giyceryl Stearate (Aldo MS)[5] | | 0.80 |
| | Oleyl Alcohol (Novol)[6] | | 0.50 |
| | Preservatives (oil soluble) | | q.s. |
| | | | 100.00 |

PROCEDURE

I. Disperse Veegum into water using high-shear mixing until smooth.

II. Add Phase B to Phase A, mixing until thoroughly dispersed.

III. Add Phase C to Phase A-B and mix until smooth.

IV. Blend Phase D and add to Phase A-B-C with gentle agitation and heat to $75° \pm 5°C$.

V. In a support vessel heat Phase E ingredients to $75° \pm 5°C$ with gentle agitation.

VI. Add Phase E to Phase A-B-C-D with gentle agitation, maintaining temperature at $75° \pm 5°C$.

VII. Maintain constant agitation and cool batch to $35° \pm 5°C$; store or fill into appropriate containers.

**"Barely There" Pressed Powder Eye Shadow**

CLE-930296

| TYPICAL PROPERTIES | |
|---------------------|---|
| COLOR | Light pink |
| ODOR | Characteristic |
| APPEARANCE | Matte |
| TEXTURE | Smooth |

(continued)

| TYPICAL PROPERTIES | |
|---|---|
| HARDNESS | $50 \pm 5$ |
| DROP TEST | Excellent |
| COVERAGE | Very good |
| SKIN ADHESION | Good |

SUPPLIERS

1. **Engelhard Corporation**
2. Whittaker, Clark & Daniels, Inc.
3. Ikeda Corporation
4. Scher Chemicals, Inc.

**"Barely There" Pressed Powder Eye Shadow**

CLE-930296

| PHASE | INGREDIENTS | | %WT. |
|---|---|---|---|
| A. | Mearitalc® TCA (Talc (and) Lauroyl Lysine)[1] | (q.s. to 100%) | 38.00 |
| | Mearlmica® SVA (Mica (and) Lauroyl Lysine)[1] | | 15.00 |
| | Zinc Stearate | | 4.00 |
| | Coslin™ C-100 (Kaolin)[1] | | 4.00 |
| | Magnesium Carbonate (Magnesium Carbonate Light USP 309)[2] | | 3.00 |
| | Silica (Spherica P-1500) | | 1.00 |
| | Cosmica® Red MCNR4 (Mica (and) Carmine)[1] | | 25.00 |
| | Preservatives | | q.s. |
| B. | Antioxidant | | q.s. |
| | isocetyl Stearate (Schercemol ICS)[4] | | 5.00 |
| C. | Flamenco® Satin Pearl 3500 (Mica (and) Titanium Dioxide)[1] | | 5,00 |
| | | | 100.00 |

PROCEDURE

I. Thoroughly blend and disperse Phase A in appropriate dry blending/dispersing equipment.

II. Spray Phase B into premixed Phase A and continue blending.

III. Pulverize and return to blender.

IV. Add Phase C to Phase A-B and mix until uniform.

**Emulsion Cream Eye Shadow**

CLE-920011

| TYPICAL PROPERTIES | |
|---|---|
| COLOR | Reddish-copper |
| ODOR | Characteristic |
| APPEARANCE | Luminous |
| TEXTURE | Creamy |
| IDENTITY | Oil/water emulsion |
| VISCOSSTY | 1,480,000 ± 14,000 cps |
| pH | 7,05 ± 0.5 |
| SPECIFIC GRAVITY | 1.25 ± 0.10 |

SUPPLIERS

1. Croda, Inc.
2. Henkel Corporation
3. ICI Surfactants
4. Amerchol Corporation
5. Stepan Company
6. **Engeihard Corporation**

**Emulsion Cream Eye Shadow**

CLE-920011

| PHASE | INGREDIENTS | | %WT. |
|---|---|---|---|
| A. | Sucrose Distearate (Crodesta F-10)[1] | | 1.10 |
| | Glycerol Monostearate (Pure) | | 5.30 |
| | Hydrogenated Coconut Oil | | 5.30 |
| | PPG-5 Lanolin Wax (Propoxyol-1695)[2] | | 1.40 |
| | Oleth-20 (Brij 98)[3] | | 1.40 |
| | Preservatives (oil soluble) | | q.s. |
| B. | Methyl Gluceth-20 (Glucam E-20)[4] | | 3.50 |
| | Lauramine Oxide (Ammonyx LO)[5] | | 0.70 |
| | Water | (q.s. to 100%) | 51.30 |
| | Preservatives (water soluble) | | q.s. |
| | Cloisonné® Rouge Flambe 440X (Mica (and) Iron Oxides (and) Titanium Dioxide)[6] | | 15.00 |
| | Duocrome® YR (Gold/Red) 422C (Mica (and) Titanium Dioxide (and) Carmine)[6] | | 15.00 |
| | | | 100.00 |

PROCEDURE

I. Add Phase B ingredients to a heated vessel and stir, bringing temperature to 75°±3°C.

II. Add Phase A ingredient to the primary vessel and heat to 75° ± 3°C, mixing until uniform.

III. Add Phase B to Phase A with constant sweep agitation.

IV. Cool gradually to 35° ± 5°C and fill.

**Pressed Powder Eyeshadow**

CLE-911036M

| TYPICAL PROPERTIES | |
|---|---|
| COLOR | Beige |
| ODOR | Characteristic |
| APPEARANCE | Pearly |
| TEXTURE | Powdery |
| HARDNESS | 45 ± 5 |
| DROP TEST | Excellent |
| COVERAGE | Excellent |
| SKIN ADHESION | Very good |

SUPPLIERS

1. **Engelhard Corporation**
2. Witco Corporation

**Pressed Powder Eyeshadow**

CLE-911036M

| PHASE | INGREDIENTS | | %WT. |
|---|---|---|---|
| A. | Talc | (q.s. to 100%) | 17.00 |
| | Zinc Stearate | | 8.00 |
| | Coslin™ C-100 (Kaolin)[1] | | 8.00 |
| | Gemtone® Tan Opal G005 (Mica (and) Iron Oxides (and) Titanium Dioxide)[1] | | 10.00 |
| | Flamenco® Ultra Sparkle 4500 (Mica (and) Titanium Dioxide)[1] | | 30.00 |
| | Preservatives | | q.s. |
| B. | Antioxidants | | q.s. |
| | Mineral Oil (Ervol White Mineral Oil)2 | | 7.00 |

(continued)

| PHASE | INGREDIENTS | %WT. |
|-------|-------------|------|
| C. | Gemtone Tan Opal G005 (Mica (and) Iron Oxides (and) Titanium Dioxide)[1] | 20.00<br>100.00 |

PROCEDURE

I. Thoroughly blend and disperse Phase A in appropriate dry blending/dispersing equipment.

II. Add Phase B ingredients into a support vessel. Heat and mix until uniform.

III. Spray Phase B into premixed Phase A and continue blending.

IV. Pulverize and return to blender.

V. Add Phase C to Phase A-B and mix until uniform.

**Charcoal Silky Powder Eyeliner**

CLE-930286M

| TYPICAL PROPERTIES | |
|--------------------|--|
| COLOR | Black |
| ODOR | Characteristic |
| APPEARANCE | Matte |
| TEXTURE | Smooth and silky |
| HARDNESS | $12 \pm 5$ |
| DROP TEST | Excellent |
| COVERAGE | Excellent |
| SKIN ADHESION | Very good |

SUPPLIERS

1. **Engelhard Corporation**
2. Witco Corporation
3. Croda, Inc.
4. Sun Chemical Corporation
5. Amerchol Corporation
6. Alzo international, Inc.

**Loose Powder Eye Shadow**

CLE-920010M

| TYPICAL PROPERTIES | |
| --- | --- |
| COLOR | Gold |
| ODOR | Characteristic |
| APPEARANCE | Luminous |
| BULK DENSITY | $13.30 \pm 0.5$ lbs/ft$^3$ |
| TEXTURE | Soft |
| COVERAGE | Excellent |
| SKIN ADHESION | Excellent |

SUPPLIERS

1. **Engelhard Corporation**
2. Witco Corporation

**Loose Powder Eye Shadow**

CLE-920010M

| PHASE | INGREDIENTS | | %WT. |
| --- | --- | --- | --- |
| A. | Talc | (q.s. to 100%) | 6.00 |
| | Zinc Stearate | | 3.00 |
| | Silica | | 1.00 |
| | Pearl-Glo® PG1085 (Bismuth Oxychloride)[1] | | 5.00 |
| | Gemtone® Amber G001 (Mica (and) Titanium Dioxide (and) Iron Oxides)[1] | | 55.00 |
| | Preservatives | | q.s. |
| B. | Mineral Oil (Carnation White Mineral Oil)[2] | | 10.00 |
| C. | Gemtone Amber G001 (Mica (and) Titanium Dioxide (and) Iron Oxides)[1] | | 20.00 |
| | | | 100.00 |

PROCEDURE

I. Thoroughly blend and disperse Phase A in appropriate dry blending/dispersing equipment.

II. Spray Phase B into premixed Phase A and continue blending.

III. Pulverize and return to blender.

IV. Add Phase C to Phase A-B and mix until uniform.

**Concealer**

CLE-910982

**PROCEDURE, *CONTINUED***

V. In a support vessel heat Phase E ingredients to 75° ± 5°C with gentle agitation.

VI. Add Phase E to Phase A-B-C-D with gentle agitation, maintaining temperature at 75° ± 5°C.

VII. Maintain constant agitation and cool batch to 35° ± 5°C; store or fill into appropriate containers.

| TYPICAL PROPERTIES | |
| --- | --- |
| COLOR | Beige |
| ODOR | Characteristic |
| APPEARANCE | Smooth |
| TEXTURE | Creamy |
| IDENTITY | oil/water emulsion |
| pH | 7.8 ± 0.5 |
| SPECIFIC GRAVITY | 1.23 ± 0.10 |
| VISCOSITY | 70,000 ± 10,000 cps |

SUPPLIERS

1, R. T. Vanderbilt Company, Inc.

2. Dow Chemical Company

3. Aqualon

4. Sun Chemical Corporation

5. **Engelhard Corporation**

6. Henkel Corporation

7. Lonza, Inc.

8. Witco Corporation

9. Amerchol Corporation

**Concealer**

CLE-910982

| PHASE | INGREDIENTS %WT. | | |
|-------|------------------|--|--|
| A. | Water | (q.s. to 100%) | 53.50 |
|    | Magnesium Aluminum Silicate (Veegum)[1] | | 2.00 |
| B. | Propylene Glycol | | 8.00 |
|    | Triethanolamine (TEA 99%)[2] | | 1.50 |
|    | Cellulose Gum (CMC-7LF)[3] | | 1.00 |
|    | Preservatives (water soluble) | | q.s. |
| C. | Titanium Dioxide | | 12.00 |
|    | Iron Oxides (C33-1700 Cosmetic Yellow)[4] | | 1.00 |
|    | Iron Oxides (C33-8098 Cosmetic Russet)[4] | | 0.50 |
|    | Iron Oxides (C33-225 Cosmetic Brown)4 | | 0.50 |
| D. | Mearimica® CF (Mica)[5] | | 6.00 |
|    | Boron Nitride | | 5.00 |
| E. | Stearic Acid (Emersol 132)[6] | | 3.00 |
|    | Glyceryl Stearate SE (Aido MSD)[7] | | 2.00 |
|    | Mineral Oil (Carnation White Mineral Oil)[2] | | 2.00 |
|    | Isopropyl Lanolate (Amerlate P)[9] | | 1.50 |
|    | Isostearic Acid (Emersol 871)[6] | | 0.50 |
|    | Preservatives (oil soluble) | | q.s. |
|    | | | 100.00 |

PROCEDURE

I. Disperse Veegum into water using high-shear mixing until smooth.

II. Add Phase B slurry to Phase A and mix until smooth.

III. Pulverize Phase C and add to Phase A-B using high-shear mixing until smooth.

IV. Add Phase D to Phase A-B-C while heating to 75° ± 5°C and mix until smooth.

*(Procedure continued on back.)*

**Sheer Satin Pressed Powder Eye Shadow**

CLE-922276

| TYPICAL PROPERTIES | |
|--------------------|--|
| COLOR | Mauve |
| ODOR | Characteristic |
| APPEARANCE | Satiny |

(continued)

| TYPICAL PROPERTIES | |
| --- | --- |
| TEXTURE | Smooth |
| HARDNESS | 42 ± 5 |
| DROP TEST | Excellent |
| COVERAGE | Very good |
| SKIN ADHESION | Good |

SUPPLIERS

1. **Engelhard Corporation**
2. Ikeda Corporation
3. ISP

**Sheer Satin Pressed Powder Eye Shadow**

CLE-922276

| PHASE | INGREDIENTS | | %WT. |
| --- | --- | --- | --- |
| A. | Mearltalc® TCA (Talc (and) Lauroyl Lysine)[1] | (q.s. to 100%) | 18.00 |
| | Mearlmica® SVA (Mica (and) Lauroy Lysine)[1] | | 20.00 |
| | Magnesium Myristate | | 5.00 |
| | Silica (Spherica P-1500)[2] | | 2.00 |
| | Cloisonné® Red 424C (Mica (and) Titanium Dioxide (and) Carmine)[1] | | 20.00 |
| | Cloisonné Violet 525C (Mica (and) Titanium Dioxide (and) Carmine (and) Ferric Ferrocyanide)[1] | | 13.00 |
| | Cloisonné Nu-Antique Blue 626CB (Mica (and) Titanium Dioxide (and) Iron Oxides (and) Ferric Ferrocyanide)[1] | | 2.00 |
| | Cloisonné Cerise Flambé 550Z (Mica (and) Iron Oxides)[1] | | 2.00 |
| | Preservatives & Antioxidant | | q.s. |
| B. | Octyl Palmitate (Ceraphyl 368)[3] | | 7.00 |
| | Isostearyl Neopentanoate (Ceraphyl 375)[3] | | 1.00 |
| | Antioxidant | | q.s. |
| C. | Cloisonné Red 424C (Mica (and) Titanium Dioxide (and) Carmine)[1] | | 5.00 |
| | Cloisonné Violet 525C (Mica (and) Titanium Dioxide (and) Carmine (and) Ferric Ferrocyanide)[1] | | 5.00 |
| | | | 100.00 |

PROCEDURE

I. Thoroughly blend and disperse Phase A in appropriate dry blending/dispersing equipment.

II. Add Phase B ingredients into a support vessel. Heat and mix until uniform.

III. Spray Phase B into premixed Phase A and continue blending.

IV. Pulverize and return to blender.

V. Add Phase C to Phase A-B and mix until uniform.

**Crayon Eye Shadow**

CLE-920009

| TYPICAL PROPERTIES | |
|---|---|
| COLOR | Sapphire |
| ODOR | Characteristic |
| APPEARANCE | Luminous |
| DROOP POINT (44°C) | Passes |
| CAPILLARY POINT | 50° ± 3°C |

SUPPLIERS

1. Strahl & Pitsch, Inc.
2. Croda, Inc.
3. Amerchol Corporation
4. Frank B. Ross Co., Inc.
5. R.I.T.A. Corporation
6. **Engelhard Corporation**

**Crayon Eye Shadow**

CLE-920009

| PHASE | INGREDIENTS | | %WT. |
|---|---|---|---|
| A. | Castor Oil | (q.s. to 100%) | 31.50 |
| | Beeswax (White Bleached Beeswax)[1] | | 4.50 |
| | Candelilla Wax (Candeliila Wax Refined Flakes)[1] | | 4.50 |
| | Myristyl Myristate (Crodamol MM)[2] | | 4.50 |
| | Octyl Palmitate | | 4.50 |
| | Isopropyl Palmitate (and) Lanolin Oil (Isopropylan 33)[5] | | 4.50 |
| | Ozokerite Wax (Ozokerite Wax 77W)[4] | | 5.00 |
| | Shea Butter (Shebu Refined)[5] | | 6.00 |
| | Isopropyl Myristate | | 3.00 |
| | Antioxidant & Preservatives | | q.s. |
| B. | Gemtone® Sapphire G011 (Mica (and) Titanium Dioxide (and) Ferric Ferrocyanide (and) Carmine)[6] | | 32.00 |
| | | | 100.00 |

PROCEDURE

I. Weigh all the ingredients in Phase A into a heated vessel and raise temperature to 85° ± 3°C, stirring until melted and uniform.

II. Add in Phase B and mix until all the pearl pigment is well dispersed.

III. Pour at 75° ± 5°C.

Note: If iron oxide or organic pigments are used, they should first be dispersed in castor oil; this mixture should then be milled in either a colloid or roller mill.

**Velvety Pressed Powder Eye Shadow**

CLE-911038M

| TYPICAL PROPERTIES | |
|---|---|
| COLOR | Green |
| ODOR | Characteristic |
| APPEARANCE | Satiny |
| TEXTURE | Creamy |
| HARDNESS | 28 ± 5 |
| DROP TEST | Fair |
| COVERAGE | Very good |
| SKIN ADHESION | Good |

SUPPLIERS

1. Sun Chemical Corporation
2. **Engelhard Corporation**
3. Witco Corporation
4. Hispano Quimica S.A./Centerchem, Inc.

**Velvety Pressed Powder Eye Shadow**

CLE-911038M

| PHASE | INGREDIENTS | | %WT. |
|---|---|---|---|
| A. | Talc | (q.s. to 100%) | 32.00 |
| | Zinc Stearate | | 3.00 |
| | Chromium Oxide Greens (C61-1245 Cosmetic Green)[1] | | 3.00 |
| | Timica® Silver Sparkle 5500 (Mica (and) Titanium Dioxide)[2] | | 11.20 |
| | Mearlmica® SVA (Mica (and) Lauroyl Lysine)[2] | | 38.80 |
| | Preservatives | | q.s. |

(continued)

| PHASE | INGREDIENTS | %WT. |
|---|---|---|
| B. | Antioxidant<br>Mineral Oil (Ervol White Mineral Oil)[3]<br>Squalane (Fitoderm)[4] | q.s.<br>7.00<br>5.00<br>100.00 |

PROCEDURE

I. Thoroughly blend and disperse Phase A in appropriate dry blending/dispersing equipment.

II. Add Phase B ingredients into a support vessel. Heat and mix until uniform.

III. Spray Phase B into premixed Phase A and mix until uniform.

IV. Pulverize and press.

**Waterproof Mascara**

CLE-910110

| TYPICAL PROPERTIES | |
|---|---|
| COLOR | Dark blue |
| ODOR | Characteristic |
| APPEARANCE | Peariescent |
| TEXTURE | Smooth |
| VISCOSITY | >2,000,000 ± 200,000 cps |
| SPECIFIC GRAVITY | 0.96 ± 0.1 |
| ADHESION | Excellent |

SUPPLIERS

1. Exxon Chemical
2. Shell Chemical Company
3. Arizona Chemical Company
4. Frank B. Ross Co., Inc.
5. Honeywell International, Inc.
6. **Engelhard Corporation**
7. Rheox, Inc.

**Waterproof Mascara**

CLE-9W110

| PHASE | INGREDIENTS | | %WT |
|---|---|---|---|
| A. | C11-12 Isoparaffin (Isopar H)[1] | (q.s. to 100%) | 45.70 |
| | Petroleum Distillate (Shell-Sol-71)[2] | | 10.00 |
| | Tall Oil Glycerides (Zonester 85)[3] | | 10.00 |
| | Carnauba Wax (#1 Yellow Carnauba Wax)[4] | | 3.00 |
| | Polyethylene (A-C Polyethylene)[5] | | 2.00 |
| B. | Talc | | 3.00 |
| | Coslin™ C-100 (Kaolin)[6] | | 3.00 |
| | Flamenco® Twilight Blue 620CB (Mica (and) | | |
| | Titanium Dioxide (and) Iron Oxides)[6] | | 10.00 |
| | Preservatives | | q.s. |
| C. | Quaternium-18 Hectorite (Bentone 38)[7] | | 10.00 |
| D. | Propylene Carbonate | | 3.30 |
| | | | 100.00 |

PROCEDURE

I. Add Phase A ingredients to a vessel equipped with a high-shear agitator; heat to $95° \pm 2°C$ with gentle agitation until a clear solution is obtained.

II. Add Phase B to Phase A and mix until uniform. Note: Care should be taken to avoid loss of volatile solvents.

III. Add Phase C to Phase A-B and mix vigorously until smooth.

IV. Add Phase D to Phase A-B-C with slow agitation; a heavy gel will form.

V. Cool batch to 60°C and fill or pump into storage drums.

**"Slender" Stick Eye Shadow**

CLE-920008

| TYPICAL PROPERTIES | |
|---|---|
| COLOR | Gray |
| ODOR | Characteristic |
| APPEARANCE | Low frost |
| DROOP POINT (44°C) | Passes |
| CAPILLARY POINT | $56° \pm 3°C$ |

SUPPLIERS

1. R.1.T.A. Corporation
2. Stepan Company
3. Amerchol Corporation
4. Roche Vitamins and Fine Chemicals
5. **Engelhard Corporation**

**"Slender" Stick Eye Shadow**

CLE-920008

| PHASE | INGREDIENTS | | %WT. |
|---|---|---|---|
| A. | Castor Oil | (q.s. to 100%) | 30.90 |
| | Shea Butter (Shebu Refined)[1] | | 6.10 |
| | Caprylic/Capric Trigiyceride (Neobee M5)[2] | | 6.10 |
| | Beeswax | | 4.60 |
| | Candelilla Wax | | 4.60 |
| | Ozokerite Wax | | 5.00 |
| | Isopropyl Myristate | | 3.00 |
| | Isopropyl Lanolate (Amerlate P)[3] | | 4.60 |
| | Talc | | 2.10 |
| | Octyl Methoxycinnamate (Parsol MCX)[4] | | 3.00 |
| | Antioxidant & Preservatives | | q.s. |
| B. | Flamenco® Twilight Blue 620CB (Mica (and) Titanium Dioxide (and) Iron Oxides)[5] | | 15.00 |
| | Gemtone® Moonstone G004 (Mica (and) Titanium Dioxide (and) Iron Oxides)[5] | | 15.00 |
| | | | 100.00 |

PROCEDURE

I. Weigh all the ingredients in Phase A into a vessel and heat to 85° ± 3°C, stirring until melted and uniform.

II. Add premixed Phase B to Phase A, maintaining temperature at 85° ± 3°C for 30 minutes with gentle agitation. (This will allow for de-aeration if vacuum is not available.)

III. Pour at 75° ± 3°C.

Note: If iron oxide or organic pigments are used, they should first be dispersed in Castor oil; this mixture should then be milled in either a colloid or roller mill.

**Silky Pressed Powder Eye Shadow**

CLE-921254

| TYPICAL PROPERTIES | |
| --- | --- |
| COLOR | Blue/violet |
| ODOR | Characteristic |
| APPEARANCE | Pearly |
| TEXTURE | Smooth |
| HARDNESS | $22 \pm 5$ |
| DROP TEST | Excellent |
| COVERAGE | Very good |
| SKIN ADHESION | Very good |

SUPPLIERS

1. Tomen America, Inc.
2. **Engelhard Corporation**
3. Dow Corning Corporation
4. Ikeda Corporation
5. Croda, Inc.
6. Exxon Chemical

**Silky Pressed Powder Eye Shadow**

CLE-921234

| PHASE | INGREDIENTS | | %WT. |
| --- | --- | --- | --- |
| A. | Talc | (q.s. to 100%) | 22.20 |
| | Zinc Stearate . | | 4.00 |
| | Polymethyl Methacrylate (Microspheres M-100)[1] | | 2.00 |
| | Cloisonné® Blue 626C (Mica (and) Titanium Dioxide (and) Ferric Ferrocyanide)[2] | | 15.68 |
| | cloisonné Violet 525C (Mica (and) Titanium Dioxide (and) Carmine (and) Ferric Ferrocyanide)[2] | | 10.12 |
| | Mearlmica®SVA (Mica (and) Lauroyl Lysine)[2] | | 10.00 |
| | Preservatives | | q.s. |
| B. | Dimethicone (Dow Corning 200 Fluid)[3] | | 9.00 |
| | Diisostearyl Malate (Cosmol 222)[4] | | 3.00 |
| | Sesame Seed Oil (Sesame Seed Oil Super Refined)[5] | | 3.00 |
| | Sorbitan Isostearate (Crill 6)[5] | | 2.50 |
| | Hydrogenated Polybutene (Panalane)[6] | | 1.50 |
| | Antioxidant | | q.s. |
| C. | Cloisonné Blue 626C (Mica (and) Titanium Dioxide (and) Ferric Ferrocyanide)[2] | | 10.00 |

(continued)

| PHASE | INGREDIENTS | %WT. |
|---|---|---|
| | Cloisonné Violet 525C (Mica (and) Titanium Dioxide (and) Carmine (and) Ferric Ferrocyanide)[2] | 7.00 |
| | | 100.00 |

## PROCEDURE

I. Thoroughly blend and disperse Phase A in appropriate dry blending/dispersing equipment.

II. Add Phase B ingredients into a support vessel. Heat and mix until uniform.

III. Spray Phase B into premixed Phase A and continue blending.

IV. Pulverize and return to blender.

V. Add Phase C to Phase A-B and mix until uniform.

**Lipstick**

CLL-980174

PROCEDURE, *CONTINUED*

III. Add in Phase C and mix with constant stirring.

IV. Pour at 75° ± 5°C.

V, Mold, cool and flame the lipsticks.

Note: If iron oxide or organic pigments are used, they should first be dispersed in Castor oil; this mixture should then be milled in either a colloid or roller mill.

| TYPICAL PROPERTIES | |
|---|---|
| COLOR | Copper |
| ODOR | Characteristic |
| APPEARANCE | Pearly |
| DROOP POINT (44°C) | Passes |
| CAPILLARY POINT | 80° ± 0.3°C |

SUPPLIERS

1. Witco Corporation

2. Croda, Inc.

3. Jeen International Corporation

4. Scher Chemicals, Inc.

5. Stepan Company

6. CREANOVA Inc.

7. Amerchol Corporation

8. Dr. Madis Laboratories, Inc.

9. Sun Chemical Corporation

10. ISP

11. **Engelhard Corporation**

**Lipstick**

CLL-980174

| PHASE | INGREDIENTS | | %WT. |
|---|---|---|---|
| A. | Candelilla Wax | | 2.75 |
| | Carnauba Wax | | 1.25 |
| | Beeswax | | 1.00 |
| | Ceresine Wax | | 5.90 |
| | Ozokerite Wax | | 6.75 |
| | Microcrystalline Wax (Multiwax 180W)[1] | | 1.40 |
| | Oleyl Alcohol (Novol)[2] | | 3.00 |
| | Isostearyl Palmitate (Jeechem ISP)[3] | | 7.50 |
| | Isostearyl Isostearate (Schercemol 1818)[4] | | 5.00 |
| | Caprylic/Capric Triglyceride (Neobee M-5)[5] | | 5.00 |
| | Bis-Diglycerylpolyalcohol Adipate-2 (Softisan 649)[6] | | 2.00 |
| | Acetylated Lanolin Alcohol (Acetulan)[7] | | 2.50 |
| | Sorbitan Tristearate (Crill 55)[2] | | 2.00 |
| | Aloe Vera (Veragel Lipoid 1:1)[8] | | 1.00 |
| | Castor Oil | (q.s. to 100%) | 57.50 |
| | Red 6 Lake (C19-008 Light Rubine Lake )[9] | | 0.25 |
| | Tocopheryl Acetate | | 0.20 |
| | Antioxidant | | q.s. |
| | Phenoxyethanol (and) Isopropylparaben, Isobutylparaben (and) Butylparaben (Liquapar PE)[10] | | 1.00 |
| B. | Cloisonné® Sparkle Copper 350J (Mica (and) Iron Oxides)[12] | | 13.00 |
| | Coslin™ C-100 (Kaolin)[11] | | 1.00 |
| C. | Fragrance | | q.s. |
| | | | 100.00 |

**PROCEDURE**

I. Weigh all the ingredients in Phase A into a heated vessel and raise temperature to 85°± 3°C, stirring until melted and uniform.

II. Add in Phase B and mix until all the pearl pigment is well dispersed.

*(Procedure continued on back.)*

Fair Taupe Lipstick

CLL-921945

| TYPICAL PROPERTIES | |
|---|---|
| COLOR | Garnet |
| ODOR | Characteristic |
| APPEARANCE | Pearlescent |
| DROOP POINT (44°C) | Passes |
| CAPILLARY POINT | 66° ± 3°C |

SUPPLIERS

1. **Engelhard Corporation**

**Fair Taupe Lipstick**

CLL-921945

| PHASE | INGREDIENTS | | %WT. |
|---|---|---|---|
| A. | Carnauba Wax | | 13.70 |
| | Beeswax | | 8.50 |
| | Isopropyl Myristate | | 30.00 |
| | Castor Oil | (q.s. to 100%) | 32.80 |
| | Antioxidant | | q.s. |
| | Preservatives | | q.s. |
| B. | Gemtone® Garnet G009 (Mica (and) Titanium Dioxide (and) Iron Oxides (and) Carmine)[1] | | 10.50 |
| | Gemtone Goldstone G0014 (Mica (and) Titanium Dioxide (and) Iron Oxides (and) Ferric Ferrocyanide)' | | 4.50 |
| C. | Fragrance | | q.s. |
| | | | 100.00 |

**PROCEDURE**

I. Weigh all the ingredients in Phase A into a heated vessel and raise temperature to 85°± 3°C, stirring until melted and uniform.

II. Add in Phase B and mix until all the pearl pigment is well dispersed.

III, Add in Phase C and mix with constant stirring.

IV. Pour at 75° ± 5°C.

V. Mold, cool and flame the lipsticks.

Note: If iron oxide or organic pigments are used, they should first be dispersed in Castor oil; this mixture should then be milled in either a colloid or roller mill.

**Lip Gloss with Sunscreen**

CLL-921972B

| TYPICAL PROPERTIES | |
| --- | --- |
| COLOR | Pink |
| ODOR | Characteristic |
| APPEARANCE | Shiny |
| ADHESION | Excellent |

SUPPLIERS

1. Croda, Inc.
2. Witco Corporation
3. Roche Vitamins and Fine Chemicals
4. Sun Chemical Corporation
5. **Engelhard Corporation**

**Lip Gloss with Sunscreen**

CLL-921972B

| PHASE | INGREDIENTS | | %WT. |
| --- | --- | --- | --- |
| A. | Castor oil | (q.s. to 100%) | 64.80 |
| | C18-36 Acid Glycol Ester (Syncrowax ERLC)[1] | | 3.70 |
| | Cetyl Alcohol (Adol 52)[2] | | 1.60 |
| | Octyl Methoxycinnamate (Parsol MCX)[3] | | 3.00 |
| | Red 30 Lake (C37-038 Permanent Pink)[4] | | 1.20 |
| | Iron Oxides (C33-1700 Cosmetic Yellow)[4] | | 0.30 |
| | Antioxidant & Preservatives | | q.s. |
| B. | Flamenco® Ultra Silk 2500 (Mica (and) Titanium Dioxide)[5] | | 10.40 |
| | Flamenco Red 420C (Mica (and) Titanium Dioxide)[5] | | 15.00 |
| C. | Fragrance | | q.s. |
| | | | 100.00 |

PROCEDURE

I. Weigh all the ingredients in Phase A into a heated vessel and raise temperature to 75° ± 5°C, stirring until melted and uniform.

II. Add Phase B ingredients to Phase A maintaining temperature at 75° ± 5°C.

III. Add Phase C and mix with constant stirring.

Note: If iron oxide or organic pigments are used, they should first be dispersed in castor oil; this mixture should then be milled in either a colloid or roller mill.

**Glossy Lipstick**

CLL-921944

| TYPICAL PROPERTIES | |
|---|---|
| COLOR | Pinkish gold |
| ODOR | Characteristic |
| APPEARANCE | Pearly |
| DROOP POINT (44°C) . | Passes |
| CAPILLARY POINT | 56° ± 0.3°C |

SUPPLIERS

1. Witco Corporation
2. Alzo International, Inc.
3. Henkel Corporation
4. Amerchol Corporation
5. R.I.T.A. Corporation
6. **Engelhard Corporation**

**Glossy Lipstick**

CLL-921944

| PHASE | INGREDIENTS | | %WT. |
|---|---|---|---|
| A. | Candelilla Wax | | 8.00 |
| | Ozokerite Wax | | 1.60 |
| | Microcrystalline Wax (Multiwax 180W)[1] | | 1.60 |
| | Octyl Palmitate (Wickenol 155)[2] | | 6.00 |
| | Cetyl Palmitate (Cutina CP)[3] | | 3.00 |
| | Lanolin Oil (Lanogene)[4] | | 18.00 |
| | Shea Butter (Shebu Butter)[5] | | 2.50 |
| | Castor Oil | (q.s. to 100%) | 44.30 |

(continued)

| PHASE | INGREDIENTS | %WT. |
|---|---|---|
|  | Antioxidant | q.s. |
|  | Preservatives | q.s. |
| B. | Cloisonné® Red 424C (Mica (and) Titanium Dioxide (and) Carmine)[6] | 5.00 |
|  | Gemtone® Sunstone G0012 (Mica (and) Titanium Dioxide iron Oxides (and) Carmine)[6] | 10.00 |
| C. | Fragrance | q.s. |
|  |  | $\overline{100.00}$ |

PROCEDURE

I. Weigh all the ingredients in Phase A into a heated vessel and raise temperature to $85° \pm 3°C$, stirring until melted and uniform.

II. Add in Phase B and mix until all the pearl pigment is well dispersed.

III. Add in Phase C and mix with constant stirring.

IV. Pour at $75° \pm 5°C$.

V. Mold, cool and flame the lipsticks.

Note: If iron oxide or organic pigments are used, they should first be dispersed in Castor oil; this mixture should then be milled in either a colloid or roller mill.

**Shimmering Brick Lipstick**

CLL-921943

| TYPICAL PROPERTIES | |
|---|---|
| COLOR | Cerise |
| ODOR | Characteristic |
| APPEARANCE | Pearlescent |
| DROOP POINT (44°C) | Passes |
| CAPILLARY POINT | $56° \pm 0.3°C$ |

SUPPLIERS

1. Amerchol Corporation
2. Witco Corporation
3. Scher Chemicals, Inc.
4. **Engelhard Corporation**

**Shimmering Brick Lipstick**

CLL-921943

| PHASE | INGREDIENTS | | %WT. |
|---|---|---|---|
| A. | Candelilla Wax | | 11.50 |
| | Beeswax | | 15.00 |
| | Isopropyl Lanolate (Amerlate P)[1] | | 15.00 |
| | isostearyl Alcohol (Witcohol 66)[2] | | 14.00 |
| | Diisopropyl Adipate (Schercemol DIA)[3] | | 13.50 |
| | Castor Oil | (q.s. to 100%) | 16.00 |
| | Antioxidant | | q.s. |
| | Preservatives | | q.s. |
| B. | Cloisonné® Super Copper 350Z (Mica (and) Iron Oxides)[4] | | 6.00 |
| | Cloisonné® Cerise Flambé 550Z (Mica (and) Iron Oxides)[4] | | 9.00 |
| C. | Fragrance | | q.s. |
| | | | 100.00 |

PROCEDURE

I. Weigh all the ingredients in Phase A into a heated vessel and raise temperature to $85° \pm 3°C$, stirring until melted and uniform.

II. Add in Phase B and mix until all the pearl pigment is well dispersed.

III. Add in Phase C and mix with constant stirring.

IV. Pour at $75° \pm 5°C$.

V. Mold, cool and flame the lipsticks.

Note: If iron oxide or organic pigments are used, they should first be dispersed in Castor oil; this mixture should then be milled in either a colloid or roller mill.

**Lip Pomade**

CLL-980209

| TYPICAL PROPERTIES | |
|---|---|
| COLOR | Hints of gold |
| ODOR | Characteristic |
| APPEARANCE | Shiny |
| ADHESION | Very good |

SUPPLIERS

1. CREANOVA Inc.
2. Stepan Company
3. Croda, Jnc.
4. Witco Corporation
5. Roche Vitamins and Fine Chemicals
6. Uniqema
7. **Engelhard Corporation**

**Lip Pomade**

CLL-980209

| PHASE | INGREDIENTS | | %WT. |
|---|---|---|---|
| A. | Hydrogenated Coco-Glycerides (Softisan 100)[1] | | 20.00 |
| | Beeswax | | 22.50 |
| | Caprylic/Capric Triglyceride (Neobee M-5)[2] | (q.s. to 100%) | 14.00 |
| | Caprylic/Capric/Stearic Triglyceride (Softisan 378)[1] | | 5.00 |
| | Cetyl Alcohol (Crodacol C-95 NF)[3] | | 5.00 |
| | Petrolatum (Super White Fonoline)[4] | | 22.85 |
| | Octyl Methoxycinnamate (Parsol MCX)[5] | | 3.00 |
| | Tocopheryl Acetate | | 0.50 |
| | Refined Milk Lipids (Monalac ML)[6] | | 3.15 |
| | Preservatives and Antioxidants | | q.s. |
| B. | Flamenco® Sparkle Gold 220J (Mica (and) Titanium Dioxide)[7] | | 4.00 |
| C. | Fragrance | | q.s. |
| | | | 100.00 |

PROCEDURE

I. Weigh all the ingredients in Phase A into a heated vessel and raise temperature to $75° \pm 5°$C, stirring until melted and uniform.

II. Add Phase B ingredients to Phase A maintaining temperature at $75° \pm 5°$C.

III. Add Phase C and mix with constant stirring.

Note: If iron oxide or organic pigments are used, they should first be dispersed in castor oil; this mixture should then be milled in either a colloid or roller mill.

**Soft Lipstick**

CLL-921 968

| TYPICAL PROPERTIES | |
|---|---|
| COLOR | Sunstone |
| ODOR | Characteristic |
| APPEARANCE | Pearlescent |
| DROOP POINT (44°C) | Passes |
| CAPILLARY POINT | 54° ± 3°C |

SUPPLIERS

1. Lipo Chemicals, Inc.
2. Croda, Inc.
3. Scher Chemicals, Inc.
4. Henkel Corporation
5. **Engelhard Corporation**

**Soft Lipstick**

CLL-921968

| PHASE | INGREDIENTS | %WT. |
|---|---|---|
| A. | Carnauba Wax | 12.00 |
| | Hydrogenated Lanolin (Lipolan)[1] | 13.00 |
| | Stearyl Heptanoate (Crodamol W)[2] | 15.00 |
| | Lauryl Lactate (Schercemol LL)[3] | 16.00 |
| | Decyl Oleate (Cetiol V)[4] | 15.00 |
| | Butyl Stearate | 14.00 |
| | Antioxidants | q.s. |
| | Preservatives | q.s. |
| B. | Flamenco® Red 420C (Mica (and) Titanium Dioxide)[5] | 7.00 |
| | Gemtone® Garnet G009 (Mica (and) Titanium Dioxide (and) Iron Oxides (and) Carmine)[5] | 2.00 |
| | Gemtone Tan Opal G005 (Mica (and) Iron Oxides (and) Titanium Dioxide)[5] | 6.00 |
| C. | Fragrance | q.s. |
| | | 100.00 |

**PROCEDURE**

I. Weigh all the ingredients in Phase A into a heated vessel and raise temperature to 85° ± 3°C, stirring until melted and uniform.

II. Add in Phase B and mix until all the pearl pigment is well dispersed.

III. Add in Phase C and mix with constant stirring.

IV. Pour at 75° ± 5°C.

V. Moid, cool and flame the lipsticks.

**Pressed Lip Powder**

CLL-921971

| TYPICAL PROPERTIES | |
| --- | --- |
| COLOR | Ruby |
| ODOR | Characteristic |
| APPEARANCE | Pearly |
| TEXTURE | Silky |
| HARDNESS | 20 ± 5 |
| DROP TEST | Excellent |
| COVERAGE | Excellent |
| SKIN ADHESION | Excellent |

SUPPLIERS

1. Sun Chemical Corporation
2. Elf Atochem North America, Inc.
3. **Engelhard Corporation**
4. Amerchol Corporation
5. Penreco
5. Dow Corning Corporation
7. Henkel Corporation,

**Pressed Lip Powder**

CLL-921971

| PHASE | INGREDIENTS | %WT. |
| --- | --- | --- |
| A. | Talc   (q.s. to 100%)   34.30 - 44.30 | |
| | Red 30 Lake (C37-038 Permanent Pink)[1] | 2.17 |
| | Titanium Dioxide (C47-056 Cosmetic White)[1] | 2.17 |
| | Nylon-12 (Orgasol 2002 Natural Cosmetic)[2] | 0.50 |
| | Preservatives | q.s. |
| | Fragrance | q.s. |
| B. | Duocrome® RY (Red/Goid) 224C (Mica (and) Titanium Dioxide (and) Carmine)[3] | 15.00 |
| | Flamenco® Ultra Silk 2500 (Mica (and) Titanium Dioxide)[3] | 10.55 |

(continued)

| PHASE | INGREDIENTS | %WT. |
|---|---|---|
| | Cloisonné® Monarch Gold 233X (Mica (and) Titanium Dioxide (and) Iron Oxides)[3] | 5.43 |
| C. | Liquid Binder* | 20.00-30.00 |
| | | 100.00 |
| | * Hydroxylated Lanolin (Ohian)[4] | 21.00 |
| | Isopropyl Niyristate | 21.00 |
| | Petrolatum (Penreco Snow White)[3] | 20.90 |
| | Isopropyl Lanolate (Amerlate P)[4] | 20.90 |
| | Dimethicone (Dow Corning 200 Fluid)[6] | 2.70 |
| | Octyl Stearate | 8.50 |
| | Octyldodecanol (Eutanol G)[7] | 5.00 |
| | Antioxidant | q.s. |
| | | 100.00 |

## PROCEDURE

I. Blend ingredients in Phase A, pulverize and return to blender.
II. Add ingredients in Phase B to Phase A and blend until uniform.
III. Melt, add and disperse premixed Phase C to premixed Phase A-B.
IV. Pulverize entire batch without screen and store.

### Long Wear Lipstick

CLL-980175F

PROCEDURE, *CONTINUED*

III. Add Phase C and mix with constant stirring.

IV. Mold, cool and flame the lipstick:

Note: If iron oxide or organic pigments are used, they should first be dispersed in Castor oil; this mixture should then be milled in either a colloid or roller mill.

| TYPICAL PROPERTIES | |
|---|---|
| COLOR | Red |
| ODOR | Characteristic |
| APPEARANCE | Pearlescent |
| DROOP POINT (44°C) | Passes |
| CAPILLARY TEST | 56°± 3°C |

SUPPLIERS

1. Amerchol Corporation

2. Wacker Silicones Corporation

3. Goidschmidt Chemica3. Corporation

4. Dow Corning Corporation

5. National Starch & Chemical Company

6. Advanced Ceramics Corporation

7. **Engelhard Corporation**

**Long Wear Lipstick**

CLL-980175F

| PHASE | INGREDIENTS | | %WT. |
|---|---|---|---|
| A. | Beeswax | | 2.00 |
| | Carnauba Wax | | 3.00 |
| | Ozokerite Wax | | 5.30 |
| | Candelilla Wax | | 5.30 |
| | Microcrystalline Wax | | 1.00 |
| | Lanolin Oil (Lanogene)[1] | | 25.00 |
| | Castor Oil | (q.s. to 100%) | 12.70 |
| | Dimethicone (and) Stearoxy Dimethylsilane (and) Dimethyldisiloxane (Belsil SDM-6022)[2] | | 5.30 |
| | Cetaryl Isononanoate (Tegosoft CI)[3] | | 15.30 |
| | Dimethicone (Dow Corning 200)[4] | | 0.50 |
| | Aluminum Starch Octenylsuccinate (Dry Flo Elite LL)[5] | | 5.00 |
| | Red 27 Lake | | 0.60 |
| | Boron Nitride (CC-6069)[6] | | 5.00 |
| | Preservatives & Antioxidants | | q.s. |
| B. | Cellini® Red 420CR7F (Mica (and) Titanium Dioxide (and) Red 7 Lake (and) Hydrogenated Polyisobutene (and) Palmitic Acid (and) Methylparaben (and) Propylparaben (and) Butylparaben)[7] | | 10.00 |
| | Flamenco® Sparkle Gold 220J (Mica (and) Titanium Dioxide)[7] | | 4.00 |
| C. | Fragrance | | q.s. |
| | | | 100.00 |

**PROCEDURE**

I. Weigh all the ingredients in Phase A into heated vessel. Heat 85° ± 5°C and stir until melted and uniform.

II. Stir in Phase B and mix slowly until all the pear! pigment is well dispersed and no air bubbles form on the surface.

*(Procedure continued on back.)*

**Lipstick with Sunscreen**

CLL-910152

| TYPICAL PROPERTIES | |
| --- | --- |
| COLOR | Bronze |
| ODOR | Characteristic |
| APPEARANCE | Pearlescent |
| DROOP POINT (44°C) | Passes |
| CAPILLARY TEST | 56° ± 3°C |

SUPPLIERS

1. Scher Chemicals, Inc.
2. Amerchol Corporation
3. Witco Corporation
4. Roche Vitamins and Fine Chemicals
5. Sun Chemical Corporation
6. **Engelhard Corporation**

**Lipstick with Sunscreen**

CLL-910152

| PHASE | INGREDIENTS | | %WT. |
| --- | --- | --- | --- |
| A. | Candelilla Wax | | 8.90 |
| | Beeswax | | 12.40 |
| | Diisopropyl Adipate (Schercemol DIA)[1] | | 13.50 |
| | Isopropyl Lanolate (Amerlate P)[2] | | 13.50 |
| | Isostearyl Alcohol (Witcohol 66)[3] | | 14.00 |
| | Castor Oil | (q.s. to 100%) | 14.73 |
| | Octyl Methoxycinnamate (Parsol MCX)[4] | | 3.00 |
| | Ozokerite Wax | | 5.10 |
| | Iron Oxides (C33-225 Cosmetic Brown)[5] | | 0.37 |
| | Antioxidant | | q.s. |
| | Preservatives | | q.s. |
| B. | Cloisonne® Rouge Flambé 440X (Mica (and) Iron Oxides (and) Titanium Dioxide)[6] | | 4.00 |
| | Duocrome® YR (Gold/Red) 422C (Mica (and) Titanium Dioxide (and) Iron Oxides)[6] | | 4.00 |
| | Gemtone® Tan Opal G005 (Mica (and) Iron Oxides (and) Titanium Dioxide)6 | | 6.50 |
| C. | Fragrance | | q.s. |
| | | | 100.00 |

**PROCEDURE**

I. Weigh all the ingredients in Phase A into heated vessel. Heat 85° ± 5°C and stir until melted and uniform.

II. Stir in Phase B and mix slowly until all the pearl pigment is well dispersed and no air bubbles form on the surface.

III. Add Phase C and mix with constant stirring.

IV. Mold, cool and flame the lipsticks.

Note: If iron oxide or organic pigments are used, they should first be dispersed in Castor oil; this mixture should then be milled in either a colloid or roller mill.

**Lip Glaze**

CLL-920052

| PHASE | INGREDIENTS | | %WT. |
|---|---|---|---|
| A. | Acetylated Lanolin (Moduian)[1] | (q.s. to 100%) | 20.60 |
| | Mineral Oil (Carnation White Mineral Oil)[2] | | 6.00 |
| | Siiica (Cab-o-sil M-5)[3] | | 2.00 |
| | Coslin™ C-100 (Kaolin)[4] | | 5.00 |
| | Red 6 Lake (C19-022 Light Rubine Lake)[5] | | 2.10 |
| | Red 30 Lake (C37-5290 Permanent Pink)[5] | | 0.40 |
| | Preservatives & Antioxidant | | q.s. |
| B. | Polybutene (Indopol H-100)[6] | | 35.00 |
| | Acetylated Lanolin (Modulan)[1] | | 18.60 |
| | Mineral Oil (Carnation White Mineral Oil)[2] | | 6.00 |
| | Ozokerite Wax (Ozokerite Wax White 77W)[7] | | 2.00 |
| C. | Cloisonne® Monarch Gold 233X (Mica (and) Titanium Dioxide (and) Iron Oxides)[4] | | 2.30 |
| D. | Fragrance | | q.s. |
| | | | 100.00 |

**PROCEDURE**

I. Add Phase A ingredients to a heated vessel equipped with a homogenizer.

II. Raise temperature to 75° ± 5°C and homogenize Phase A for 30 minutes until smooth.

III. Add Phase B ingredients to Phase A, maintaining temperature at 75° ± 5°C and agitating with a marine type mixer.

IV. When the Ozokerite Wax has melted and product is uniform, add Phase C ingredients and mix an additional 15 minutes.

V. Add Phase D and mix with constant stirring.

VI. Cool to 40° ± 5°C and fill into containers.

Note: If iron oxide or organic pigments are used, they should first be dispersed in mineral oil; this mixture should then be milled in either a colloid or roller mill.

**Lip Glaze**

CLL-920052

| TYPICAL PROPERTIES | |
|---|---|
| COLOR | Bronze |
| ODOR | Characteristic |
| APPEARANCE | Wet |
| SPECIFIC GRAVITY | 0.93 ± 0.1 |

SUPPLIERS

1. Amerchol Corporation
2. Witco Corporation
3. Cabot Corporation
4. **Engelhard Corporation**
5. Sun Chemical Corporation
6. Amoco Chemicals
7. Frank B. Ross Co., Inc.

**Face Bronzer with Sunscreen**

CLF-920027M

| PHASE | INGREDIENTS | | %WT. |
|---|---|---|---|
| A. | Water | (q.s. to 100%) | 52.90 |
| | Carbomer | | 0.50 |
| B. | DMDM Hydantoin (Glydant)[1] | | 0.40 |
| | Dehydroacetic Acid | | 0.10 |
| | Sodium Hydroxide (40%) | | 0.40 |
| | Glycerin | | 5.00 |
| C. | Stearyl Alcohol (and) Ceteareth-20 (Promulgen G)[2] | | 2.20 |
| | Glyceryl Stearate (and) PEG-100 Stearate (Arlacel 165)[3] | | 5.00 |
| | Cetyl Alcohol | | 0.60 |
| | Isopropyl Isostearate | | 3.90 |
| | Mineral Oil | | 7.50 |
| | Myristyl Lactate | | 0.50 |
| | Octyl Methoxycinnamate | | 7.00 |
| | Benzophenone-3 | | 4.00 |
| D. | Cloisonné® Sparkle Gold 222J (Mica (and) Titanium Dioxide (and) Iron Oxides)[4] | | 5.00 |

(continued)

| PHASE | INGREDIENTS | %WT. |
|---|---|---|
| | Dimethicone (and) Trimethylsiloxysilicate (SS-4267)[5] | 3.00 |
| | Cyclic Silicones (and) Dimethyl Gum Fluid (SF-1214)[5] | 2.00 |
| | | 100.00 |

**PROCEDURE**

I. Add water to a vessel equipped with a high-shear agitator. Heat to 75° ± 3°C and add Carbomer, mixing until dispersed.

II. Add Phase B to Phase A and continue mixing, maintaining temperature at 75°±3°C.

III. Add Phase C to support vessel and heat to 75° ± 3°C using moderate agitation.

*(Procedure continued on back.)*

**Face Bronzer with Sunscreen**

CLF-920027M

PROCEDURE, *CONTINUED*

IV. Add Phase C to Phase A-B maintaining temperature and using constant agitation.
V. Cool to 55° ± 5°C and add Phase D ingredients.
VI. Continue mixing and cool to 25°C. Pass through a colloid mill and store.

| TYPICAL PROPERTIES | |
|---|---|
| COLOR | Gold ivory |
| ODOR | Characteristic |
| APPEARANCE | Sparkly |
| TEXTURE | Thick - creamy |
| pH | 7.5 ± 0.2 |
| SPECIFIC GRAVITY | 1.0 |

SUPPLIERS

1. Lonza, Inc.

2. Amerchol Corporation

3. ICI Surfactants

4. **Engelhard Corporation**

5. GE Silicones

**Sheer Leg Makeup**

CLB-920046

| PHASE | INGREDIENTS | %WT. |
|---|---|---|
| A. | Petroleum Distillate, (and) Quaternium-18 Hectorite (and) Propylene Carbonate (Bentone Gel SS-71)[1] (q.s. to 100%) | 41.80 |
| B. | C9-11 Isoparaffin (Soltrol 100)[2] | 3.00 |
| | Cyclomethicone (Silicone SF-1173)[3] | 3.00 |
| C. | Beeswax (Beeswax White Refined Bleached)[4] | 10.00 |
| | PVP/Eicosene Copolymer (Ganex V-220)[5] | 5.00 |
| | Stearyl Alcohol (Crodacol S-95)[6] | 3.00 |
| | Isopropyl Lanolate (Amerlate P)[7] | 2.00 |
| | Stearic Acid (Emersol 132)[8] | 1.00 |
| | Silica | 0.80 |
| | Triethanolamine | 0.50 |
| | Preservatives | q.s. |
| D. | Gemtone® Tan Opal G005 (Mica (and) Iron Oxides (and) Titanium Dioxide)[9] | 10.00 |
| | Flamenco® Superpearl 120C (Mica (and) Titanium Dioxide)[9] | 15.00 |
| | Mearlmica® SVA (Mica (and) Lauroyi Lysine)[9] | 4.90 |
| | | 100.00 |

PROCEDURE

I. Add Phase A ingredient to a vessel equipped with a side sweep agitator.
II. Mix in Phase B with gentle agitation until smooth. Heat to 75°C.
III. Premix Phase C ingredients and heat to 75°C. Add Phase C to Phase A-B.
IV. Disperse pigment (Phase D) into hot base. Mix until completely uniform.
V. Cool and pour into appropriate packaging.

Note: Care should be taken to avoid loss of volatile solvents.

**Sheer Leg Makeup**

CLB-920046

| TYPICAL PROPERTIES | |
|---|---|
| COLOR | Suntan |
| ODOR | Characteristic |
| APPEARANCE | Sheer |
| TEXTURE | Smooth paste |
| IDENTITY | Anhydrous cream |

(continued)

| TYPICAL PROPERTIES | |
|---|---|
| SPECIFIC GRAVITY | $1.03 \pm 0.10$ |

SUPPLIERS

1. Rheox, Inc.
2. Phillips 66 Company
3. GE Silicones
4. Frank B. Ross Co., Inc.
5. ISP
6. Croda, Inc.
7. Amerchol Corporation
8. Henkel Corporation
9. **Engelhard Corporation**

**Creamy Pearl Blush**

CLF-921236

| PHASE | INGREDIENTS | | %WT. |
|---|---|---|---|
| A. | Laneth-16 (and) Ceteth-16 (and) Oleth 16 (and) Steareth 16 (Solulan 16)[1] | | 2.20 |
| | Isopropyl Myristate | | 2.70 |
| | Beeswax (Beeswax Synthetic)[2] | | 2.70 |
| | Sorbitan Monostearate (Arlacel 60)[3] | | 5.50 |
| | Distilled Lanolin Alcohol (Super Hartolan)[4] | | 5.30 |
| | Glyceryl Tribehenate (Syncrowax HR-C)[4] | | 3.90 |
| | Paraffin | | 4.40 |
| | Preservatives (oil soluble) & Antioxidant | | q.s. |
| B. | Propylene Glycol | | 5.40 |
| | Polysorbate 60 (Tween 60)[3] | | 2.60 |
| | Water | (q.s. to 100%) | 45.40 |
| | Preservatives (water soluble) | | q.s. |
| C. | Cloisonné® Red 424C (Mica and Titanium Dioxide (and) Carmine)[5] | | 6.70 |
| | Cloisonné Rouge Flambe 440X (Mica (and) Iron Oxides (and) Titanium Dioxide)5 | | 6.60 |
| | Flamenco® Superpearl 120C (Mica (and) Titanium Dioxide)[5] | | 6.60 |
| D. | Fragrance | | q.s. |
| | | | 100.00 |

**PROCEDURE**

I. Heat Phase A to $85° \pm 3°C$ with stirring until all ingredients are melted.

II. In a separate vessel heat Phase B to 85°± 3°C with stirring until fully dispersed.

III. Add Phase C to Phase B with stirring until fully dispersed and uniform.

*(Procedure continued on back.)*

**Creamy Pearl Blush**

CLF-921236

PROCEDURE, *CONTINUED*

IV. Add Phase A to combined Phase B-C with stirring and remove from heat.
V. Add Phase D with stirring at 43° ± 3°C.
VI. Drop batch below 30°C and fill at room temperature.

| TYPICAL PROPERTIES | |
|---|---|
| COLOR | Reddish-copper |
| ODOR | Characteristic |
| APPEARANCE | Luminous |
| TEXTURE | Creamy |
| IDENTITY | Oil/water emulsion |
| VISCOSITY | >2,000,000 ± 200,000 cps |
| pH | 6.2 ± 0.5 |
| SPECIFIC GRAVITY | 1.10 ± 0.1 |

SUPPLIERS

1. Amerchol Corporation
2. Frank B. Ross Co., Inc.
3. ICI Surfactants
4. Croda, Inc.
5. **Engelhard Corporation**

**Liquid Foundation**

CLF-940152A

| PHASE | INGREDIENTS | | %WT. |
|---|---|---|---|
| A. | Xanthan Gum (Keltrol T)[1] | | 0.20 |
| | Cellulose Gum (CMC 7LF)[2] | | 0.20 |
| | Water | (q.s. to 100%) | 69.31 |
| B. | Triethanolamine (TEA 99%)[3] | | 0.65 |
| | PEG-7 Glyceryl Cocoate (Cetiol HE)[4] | | 6.00 |
| | Preservatives (water soluble) | | q.s. |

(continued)

| PHASE | INGREDIENTS | %WT. |
|---|---|---|
| C. | Iron Oxides (C33-210 Cosmetic Yellow)[5] | 1.20 |
| | Talc | 0.75 |
| | Cloisonné® Satin Rouge 450M (Mica (and) Iron Oxides)[6] | 0.60 |
| | Flamenco® Satin Pearl 3500 (Mica (and) Titanium Dioxide)[6] | 1.49 |
| | Cloisonné Satin Bronze 250M (Mica (and) Iron Oxides)[6] | 3.60 |
| D. | Isopropyl Myristate | 2.00 |
| | Oleyl Alcohol (Novol)[7] | 6.50 |
| | Mineral Oil (and) Lanolin Alcohol (Amerchol L-101)[8] | 4.50 |
| | Cetearyl Alcohol (Lanette O)[4] | 2.00 |
| | Stearic Acid | 1.00 |
| | Preservatives (oil soluble) | q.s. |
| | | 100.00 |

## PROCEDURE

I. Disperse gums into water using high-shear mixing until smooth.

II. Add Phase B to Phase A and mix until smooth.

III. Pulverize Phase C and add to Phase A-B using high-shear mixing until smooth.

IV. In a support vessel heat Phase D ingredients to $75° \pm 5°C$ with gentle agitation.

V. Add Phase D to Phase A-B-C- with gentle agitation, maintaining temperature at $75° \pm 5°C$.

VI. Maintain constant agitation and cool batch to $35° \pm 5°C$; store or fill into appropriate containers.

**Liquid Foundation**

CLF-940152A

| TYPICAL PROPERTIES | |
|---|---|
| COLOR | Suntan beige |
| ODOR | Characteristic |
| APPEARANCE | Smooth |
| TEXTURE | Creamy |
| IDENTITY | Oil/water emulsion |
| pH | $8.00 \pm 0.5$ |
| SPECIFIC GRAVITY | $1.03 \pm 0.100$ |
| VISCOSITY | $680 \pm 60$ cps |

SUPPLIERS

1. The NutraSweet Kelco Company
2. Aqualon
3. Dow Chemical Company
4. Henkel Corporation
5. Sun Chemical Corporation
6. **Engelhard Corporation**
7. Croda, Inc.
8. Amerchol Corporation

**Earthtone Makeup Powder**

CLF-920026

| PHASE | INGREDIENTS | | %WT. |
|---|---|---|---|
| A. | Talc | (q.s. to 100%) | 27.30 |
| | Iron Oxides (C33-2199 Cosmetic Russet)[1] | | 27.30 |
| | Preservatives | | q.s. |
| | Bi-Lite® Ultralite BL3186 (Mica (and) Bismuth Oxychloride)[2] | | 27.30 |
| | Cloisonné® Imperial Gold 222X (Mica (and) Titanium Dioxide (and) Iron Oxides)[2] | | 9.10 |
| B. | Mineral Oil (Ervol White Mineral Oil)[3] | | 4.50 |
| | Isopropyl Myristate | | 4.50 |
| | Fragrance | | q.s. |
| | | | 100.00 |

**PROCEDURE**

I. Thoroughly blend and disperse Phase A in appropriate dry blending/dispersing equipment.

II. Blend Phase B ingredients until uniform.

III. Spray Phase B into premixed Phase A and continue blending.

IV. Pulverize and store.

**Earthtone Makeup Powder**

CLF-920026

| TYPICAL PROPERTIES | |
|---|---|
| COLOR | Burgundy |
| ODOR | Characteristic |
| APPEARANCE | Matte |

(continued)

| TYPICAL PROPERTIES | |
| --- | --- |
| TEXTURE | Smooth |
| BULK DENSITY | $19,7 \pm 0.5$ lbs/ft$^3$ |
| COVERAGE | Excellent |
| SKIN ADHESION | Excellent |

SUPPLIERS

1. Sun Chemical Corporation
2. **Engelhard Corporation**
3. Witco Corporation

**Body Veil**

CLB-980035

| PHASE | INGREDIENTS | | %WT. |
| --- | --- | --- | --- |
| A. | Water | (q.s. to100%) | 29.30 |
| | Carbomer (Carbopol 941)[1] | | 0.25 |
| | Alcohol SD-40 | | 30.00 |
| B. | 1,3 Butylene Glycol | | 8.00 |
| | Flamenco® Satin Gold 260M (Mica (and) | | |
| | Titanium Dioxide (and) Iron Oxides)[2] | | 2.00 |
| | Flamenco Sparkle Gold 220J (Mica (and) Titanium Dioxide)[2] | | 5.00 |
| C. | Glyceryl Polyacrylate (and) Glycerine (and) | | |
| | Water (Hispagel 200)[3] | | 14.00 |
| | Squalane (Fitoderm)[3] | | 2.00 |
| | Cellini® Coral 420CR40F (Mica (and) Titanium Dioxide (and) Red 40 Lake (and) Hydrogenated Polyisobutene (and) Palmitic Acid (and) Methylparaben (and) Propylparaben (and) Butylparaben)[2] | | 0.10 |
| D. | Water | | 1.00 |
| | Triethanolamine | | 0.25 |
| E. | PVP (PVP K-30) (20% aqueous solution)[4] | | 10.00 |
| | Fragrance (Herbal 11143V)[5] | | 0.10 |
| | | | 100.00 |

**PROCEDURE**

I. Using moderate propeller agitation sprinkle the carbomer into the water and allow to mix until fully hydrated, then add the alcohol.

II. Add premixed Phase B to Phase A.

III. In a separate vessel weigh out the Hispagel 200. Add Phase A-B ingredients to the Hispagel 200 with gentle mixing. Add the rest of Phase C ingredients.

IV. Add premixed Phase D to Phase A-B-C.

V. Add Phase E to Phase A-B-C-D.

**Body Veil**

CLB-980035

| TYPICAL PROPERTIES | |
|---|---|
| COLOR | Translucent gel with orange sparkles |
| TEXTURE | Silky gel |
| IDENTITY | Gel |
| VISCOSITY | 280,000 ± 5,000 cps |
| pH | 7.50 ± 0.50 |

SUPPLIERS

1. BF Goodrich Specialty Chemicals
2. **Engelhard Corporation**
3. Hispano Quimica S.A./Centerchem, Inc.
4. ISP
5. Shaw Mudge & Company

**Liquid Makeup**

CLF-921084

| PHASE | INGREDIENTS | | %WT. |
|---|---|---|---|
| A. | Mineral Oil (Carnation White Mineral Oil)[1] | | 4.50 |
| | Mineral Oil (and) Lanolin Alcohol (Amerchol L-101)[2] | | 4.50 |
| | Stearic Acid (Emersol 132)[3] | | 2.20 |
| | Glyceryl Stearate (Arlacel 125)[4] | | 1.80 |
| | Isopropyl Lanolate (Amerlate P)[2] | | 0.90 |
| | Isostearic Acid (Emersol 871)[3] | | 0.50 |
| | Preservatives (oil soluble) | | q.s. |
| B. | Water | (q.s. to 100%) | 50.20 |
| | Propylene Glycol | | 4.50 |
| | Triethanolamine (TEA 99%)[5] | | 0.90 |
| | Preservatives (water soluble) | | q.s. |
| C. | Titanium Dioxide (C47-056 Cosmetic White)[6] | | 2.00 |
| | Iron Oxides (C33-225 Cosmetic Brown)[6] | | 0.20 |
| | Talc | | 7,80 . |
| D. | Mearlmaid® AA (Water (and) Guanine (and) Isopropyl | | |

(continued)

| PHASE | INGREDIENTS | %WT. |
|-------|-------------|------|
| | Alcohol (and) Methylcellulose)[7] | 10.00 |
| | Water | 10.00 |
| | | 100.00 |

## PROCEDURE

I. Heat Phase A to 85° ± 3°C while mixing until completely uniform.

II. Heat Phase B to 85° ± 3°C while mixing.

III. Pulverize Phase C and add to Phase B using high-shear mixing until smooth.

IV. Add Phase A to Phase B-C maintaining constant agitation and cool batch to 35° ± 5°C.

V. Add premixed Phase D to Phase A-B-C with gentle agitation while mixing until completely uniform.

VI. Store or fill into appropriate containers.

**Liquid Makeup**

CLF-921084

| TYPICAL PROPERTIES | |
|--------------------|--|
| COLOR | Pearly beige |
| ODOR | Characteristic |
| APPEARANCE | Smooth |
| TEXTURE | Creamy |
| IDENTITY | Oil/water emulsion |
| pH | 7.7± 0.5 |
| SPECIFIC GRAVITY | 1.05 ± 0.100 |
| VISCOSITY | 10,000 ± 1,000 cps |

SUPPLIERS

1. Witco Corporation
2. Amerchol Corporation
3. Henkel Corporation
4. ICI Surfactants
5. Dow Chemical Company
6. Sun Chemical Corporation
7. **Engelhard Corporation**

**Silky Finish Loose Face Powder**

CLF-950038

| PHASE | INGREDIENTS | | %WT. |
|---|---|---|---|
| A. | Mearlcite[TM] SRA (Mica (and) Lauroyl Lysine)[1] | (q.s. to 100%) | 47.60 |
| | Mearimica® SVA (Mica (and) Lauroyl Lysine)[1] | | 30.75 |
| | Zinc Stearate | | 3.00 |
| | Silk Powder (Crosilk Powder)[2] | | 2.00 |
| | Magnesium Carbonate (Light USP 309)[3] | | 2.00 |
| | Boron Nitride | | 2.00 |
| | Iron Oxides (C33-1700 Cosmetic Yellow)[4] | | 0.30 |
| | Iron Oxides (C33-225 Cosmetic Brown)[4] | | 0.10 |
| | Red 30 Lake (C37-5290 Permanent Pink)[4] | | 0.20 |
| | Flamenco® Orange 320C (Mica (and) Titanium Dioxide)[7] | | 4.25 |
| | Cosmica® Orange MCR101 (Mica (and) Iron Oxides)[7] | | 3.30 |
| | Preservatives | | q.s. |
| B. | Caprylic/Capric Triglycerides (Neobee M-5)[5] | | 2.25 |
| | Phenyl Trimethicone (Dow Corning 556 Fluid)[6] | | 1.75 |
| | Tocopherol Acetate | | 0.50 |
| | Fragrance | | q.s. |
| | | | 100.00 |

<u>PROCEDURE</u>

I. Thorough blend and disperse Phase A in appropriate dry blending/dispersing equipment.

II. Blend Phase B ingredients until uniform.

III. Spray Phase B into premixed Phase A and continue blending.

IV. Pulverize and store.

**Silky Finish Loose Face Powder**

CLF-950038

| TYPICAL PROPERTIES | |
|---|---|
| COLOR | Peach |
| ODOR | Characteristic |
| APPEARANCE | Matte |
| TEXTURE | Smooth |
| BULK DENSITY | $10.3 \pm 0.5$ lbs/ft$^3$ |
| COVERAGE | Very good |
| SKIN ADHESION | Very good |

SUPPLIERS

1. **Engelhard Corporation**
2. Croda, Inc.
3. Whittaker, Clark & Daniels, Inc.
4. Sun Chemical Corporation
5. Stepan Company
6. Dow Corning Corporation

**Face Bronzer Pressed Powder**

CLF-921235M

| PHASE | INGREDIENTS | | %WT. |
|-------|-------------|---|------|
| A. | Talc | (q.s. to 100%) | 38.70 |
| | Zinc Stearate | | 6.00 |
| | Iron Oxides (C33-215 Cosmetic Russet)[1] | | 8.80 |
| | Iron Oxides (C33-225 Cosmetic Brown)[1] | | 6.50 |
| | Iron Oxides (C33-1700 Cosmetic-Yellow)[1] | | 3.50 |
| | Iron Oxides (C33-4799 Cosmetic Black)[1] | | 0.90 |
| | Iron Oxides (C33-8098 Cosmetic Russet)[1] | | 0.90 |
| | Cloisonné® Gold 222C (Mica (and) Titanium Dioxide (and) Iron Oxides)[2] | | 1.00 |
| | Flamenco® Velvet 120V (Mica (and) Titanium Dioxide)[2] | | 6.50 |
| | Bi-Lite® 20 (Mica (and) Bismuth Oxychloride)[2] | | 21.20 |
| | Preservatives | | q.s. |
| B. | Fragrance | | q.s. |
| | Antioxidant | | q.s. |
| | Mineral Oil | | 3.50 |
| | Octyl Methoxycinnamate (Parsol MCX)[3] | | 2.50 |
| | | | 100.00 |

**PROCEDURE**

I. Thoroughly blend and disperse Phase A in appropriate dry blending/dispersing equipment.

II. Add Phase B ingredients into a support vessel. Heat and mix until uniform.

III. Spray Phase B into premixed Phase A and continue blending.

IV. Pulverize and press.

**Face Bronzer Pressed Powder**

CLF-921235M

| TYPICAL PROPERTIES | |
| --- | --- |
| COLOR | Reddish brown |
| ODOR | Characteristic |
| APPEARANCE | Matte |
| TEXTURE | Silky |
| HARDNESS | $40 \pm 5$ |
| DROP TEST | Good |
| COVERAGE | Excellent |
| SKIN ADHESION | Excellent |

SUPPLIERS

1. Sun Chemical Corporation
2. **Engelhard Corporation**
3. Roche Vitamins and Fine Chemicals

**Blush Stick**

CLF-970266

| PHASE | INGREDIENTS | | %WT. |
| --- | --- | --- | --- |
| A. | Isostearyl Neopentanoate (Schercemol 185)[1] | | 33.70 |
| | Talc | (q.s. to 100%) | 15.30 |
| | Ozokerite Wax | | 13.00 |
| | Castor Oil | | 11.00 |
| | Candelilla Wax | | 4.00 |
| | Octyl Methoxycinnamate (Parsol MCX)[2] | | 3.00 |
| | Antioxidant & Preservatives | | q.s. |
| B. | Duocrome® RY (Red/Gold) 224C (Mica (and) Titanium Dioxide (and) Carmine)[3] | | 15.00 |
| | Cellini® Coral 420CR40F (Mica (and) Titanium Dioxide (and) Red 40 Lake (and) Hydrogenated Polyisobutene (and) Palmitic Acid (and) Methylparaben (and) Propylparaben (and) Butylparaben)[3] | | 5.00 |
| C. | Fragrance | | q.s. |
| | | | 100.00 |

## PROCEDURE

I. Weigh all the ingredients in Phase A into a heated vessel. Heat and stir until melted and uniform.

II. Add Phase B maintaining temperature at 75° ± 3°C and mix until ail the pigment is well dispersed.

III. Add Phase C and mix with constant stirring.

IV. Mold into sticks.

Note: If iron oxide or organic pigments are used, they should first be dispersed in castor oil; this mixture should then be milled in either a colloid or roller mill.

**Blush Stick**

CLF-970266

| TYPICAL PROPERTIES | |
| --- | --- |
| COLOR | Pink |
| ODOR | Characteristic |
| APPEARANCE | Pearly |
| DROOP POINT (44°C) | Passes |
| CAPILLARY POINT | 55° ± 3°C |

SUPPLIERS

1. Scher Chemicals, Inc.
2. Roche Vitamins and Fine Chemicals
3. **Engelhard Corporation**

**Pearly Foundation Cream**

CLF-921966

| PHASE | INGREDIENTS | %WT. |
| --- | --- | --- |
| A. | Polysorbate 80 (and) Cetyl Acetate (and) Acetylated Lanolin Alcohol (Solulan 98)[1] | 3.00 |
| | Isopropyl Lanolate (Amerlate P)[1] | 5.50 |
| | Acetylated Lanolin Alcohol (Acetulan)[1] | 5.30 |
| | Glyceryl Stearate SE (Tegin G)[2] | 3.50 |
| | Stearic Acid | 2.70 |
| | Preservatives (oil soluble) | q.s. |
| | Antioxidants | q.s. |
| B. | Propylene Glycol | 5.00 |
| | Triethanolamine | 1.00 |

(continued)

| PHASE | INGREDIENTS | | %WT. |
|---|---|---|---|
| | Water | (q.s. to 100%) | 64.00 |
| | Preservatives (water soluble) | | q.s. |
| | Fragrance | | q.s. |
| C. | Flamenco® Ultra Silk 2500 (Mica (and) Titanium Dioxide)[3] | | 9.00 |
| | Cloisonné® Super Bronze 2500 (Mica (and) Iron Oxides)[3] | | 1.00 |
| | | | 100.00 |

## PROCEDURE

I. Heat Phases A and B in separate vessels to 80° ± 3°C

II. Mix Phase B into Phase A. Then cool to 40° ± 3°C. Add Phase C and mix until the pigment is well dispersed.

III. Continue mixing and fill.

**Pearly Foundation Cream**

CLF-921966

| TYPICAL PROPERTIES | |
|---|---|
| COLOR | Tan. |
| ODOR | Characteristic |
| APPEARANCE | Pearly |
| TEXTURE | Creamy |
| IDENTITY | Oil/water emulsion |
| VISCOSITY | 13,700 ± 1,000 cps |
| pH | 7.7 ± 0.5 |
| SPECIFIC GRAVITY | 1.1 ± 0.1 |

SUPPLIERS

1. Amerchol Corporation
2. Goldschmidt Chemical Corporation
3. **Engelhard Corporation**

**Conditioning Shampoo**

CLH-940275M

| PHASE | INGREDIENTS | | %WT. |
|---|---|---|---|
| A. | Water | (q.s. to 100%) | 64.43 |

(continued)

| PHASE | INGREDIENTS | %WT. |
|---|---|---|
| | Xanthan Gum (Keltrol)[1] | q.s. |
| | Starch/Acrylates/Acrylamide Copolymer (Water-Lock D212)[2] | 0.10 |
| | Preservatives | 0.60 |
| B. | TEA-Lauryl Sulfate (Standapol T)[3] | 25.00 |
| | Lauramide DEA (Monamid 716)[4] | 3.50 |
| C. | Cloisonné® Sparkle Gold 222J (Mica (and) Titanium Dioxide (and) Iron Oxides)[5] | 0.05 |
| | Water | 5.00 |
| | Panthenol (DL-Panthenol)[6] | 0.80 |
| | Green 3 (0.5% aqueous solution) | 0.02 |
| | Fragrance (TF-157146 Floral Fruity Bouquet)[7] | 0.50 |
| | Citric Acid (20% solution) | q.s. |
| | | 100.00 |

## PROCEDURE

I. Disperse preservatives, Copolymer and Xanthan gum into de-ionized water with high-shear agitation.

II. Add Phase B ingredients in listed order to Phase A and mix until uniform.

III. Add premixed Phase C to Phase A-B with gentle agitation.

IV. Adjust pH with Citric Acid.

**Conditioning Shampoo**

CLH-940275M

| TYPICAL PROPERTIES | |
|---|---|
| COLOR | Light green with gold sparkle |
| TEXTURE | Characteristic |
| IDENTITY | Lustrous |
| VISCOSITY | 5,454 ± 500 cps |
| pH | 6.7 ±0.5 |
| SPECIFIC GRAVITY | 1.01 ± 0.10 |

## SUPPLIERS

1. The NutraSweet Kelco Company
2. Grain Processing Corporation
3. Henkel Corporation
4. Uniqema
5. **Engelhard Corporation**

6. Tri-K Industries
7. Technology, Flavors & Fragrances, Inc.

**Iridescent Hair Conditioner**

CLH-973019B

| PHASE | INGREDIENTS | %WT. | |
|-------|-------------|------|------|
| A. | Cyclomethicone (and) Dimethiconol (and) Dimethicone (Abil OSW-12)[1] | | 27.00 |
| | Phenyl Trimethicone (Abil AV 20)[1] | | 4.50 |
| | Propylene Glycol | | 31.00 |
| | Methylchloroisothiazolinone (and) Methylisothiazolinone (Kathon CG)[2] | | 0.05 |
| | Fragrance 16148M[3] | | 0.30 |
| B. | Water | (q.s. to 100%) | 34.35 |
| | Ext. Violet 2 (0.10% aqueous solution) | | 0.25 |
| | Flamenco® Ultra Sparkle 4500 (Mica (and) Titanium Dioxide)[4] | | 0.30 |
| C. | Polyacrylamide (and) C13-14 Isoparaffin (and) Laureth-7 (Sepigel 305)[5] | | 2.25 |
| | | | 100.00 |

## PROCEDURE

I. Using moderate propeller agitation, add ingredients of the Phase A in the order listed, at room temperature.
II. Add the premixed Phase B with mixing.
III. Add Phase C and mix until uniform.

**Iridescent Hair Conditioner**

CLH-973019B

| TYPICAL PROPERTIES | |
|--------------------|------|
| COLOR | Soft lavender with silver sparkle |
| TEXTURE | Silky gel |
| IDENTITY | Water in silicone gel |
| VISCOSITY | 25,000 ± 2,000 cps |
| pH | 6.50 ± 0.50 |

## SUPPLIERS

1. Goldschmidt Chemical Corporation
2. Rohm and Haas Company, Inc.

3. Shaw Mudge & Company
4. **Engelhard Corporation**
5. Seppic

**Festival Hair Gel**

CLH-992002B

| PHASE | INGREDIENTS | | %WT. |
|---|---|---|---|
| A. | Water | (q.s. to 100%) | 79.15 |
| | Carbomer (Carbopol Ultrez 10)[1] | | 0.50 |
| B. | Propylene Glycol | | 4.00 |
| | Preservatives | | q.s. |
| C. | Triethanolamine | | 0.50 |
| D. | Water | | 15.00 |
| | PVP | | 0.20 |
| | Disodium EDTA | | 0.05 |
| | Reflecks™ Gleams of Gold G230L (Calcium Sodium Borosilicate (and) Titanium Dioxide)[2] | | 0.15 |
| | Benzophenone-4 (Uvinul MS-40)[3] | | 0.05 |
| E. | Polysorbate 20 (Tween 20)[4] | | 0.20 |
| | Fragrance (CK TYPE#18567H)[5] | | 0.10 |
| | Ext. Red 4 (0.5% aqueous solution) | | 0.10 |
| | | | 100.00 |

## PROCEDURE

I. Combine Phase A ingredients and mix until thoroughly dispersed.
II. Add premixed Phase B to Phase A while mixing until completely uniform.
III. Add Phase C to Phase A-B while under agitation.
IV. Heat premixed Phase D until PVP is dissolved and add to Phase A-B-C.
V. Add premixed Phase E to Phase A-B-C-D.

**Festival Hair Gel**

CLH-992002B

| TYPICAL PROPERTIES | |
|---|---|
| COLOR | Pink |
| ODOR | Characteristic |
| APPEARANCE | Luminous |
| VISCOSITY | 18,000 ± 2,000 cps |
| pH | 6.5 ± 0.5 |

SUPPLIERS

1. BF Goodrich Specialty Chemicals
2. **Engelhard Corporation**
3. BASF Corporation
4. ICI America
5. Shaw Mudge & Company

**Sparkle Hair Spray**

CLH-982984B

| PHASE | INGREDIENTS | | %WT. |
|---|---|---|---|
| A. | Water | (q.s. to 100%) | 95.85 |
| | PVM/MA Decadiene Crosspolymer (Stabileze 06)[1] | | 0.25 |
| | PVP (PVP K-30)[1] | | 2.00 |
| | Sodium Hydroxide (10% aqueous solution) | | 0.25 |
| | Methylchloroisothiazolinone (and) | | |
| | Methylisothiazolinone (Kathon CG)[2] | | 0.05 |
| | Sodium Hydroxide (10% aqueous solution) | | 0.15 |
| B. | Propylene Glycol | | 1.00 |
| | Cloisonné® Sparkle Gold 222J (Mica (and) Titanium | | |
| | Dioxide (and) Iron Oxides)[3] | | 0.10 |
| | Yellow 8 (0.5% aqueous solution) | | 0.10 |
| | Green 3 (0.5% aqueous solution) | | 0.05 |
| C. | Fragrance (CK-One Type 18567H SMCO)[4] | | 0.10 |
| | Octoxynol-9 | | 0.10 |
| | | | 100.00 |

**PROCEDURE**

I. Disperse Stabileze in water at room temperature with mixing. Start heating to 70°C and mix until uniform.

II. Add PVP at 60°C and mix while cooling.

III. Add the rest of the ingredients of Phase A at 45°C with mixing.

IV. At 35°C add the premixed Phase B and Phase C ingredients. Mix until uniform.

**Sparkle Hair Spray**

CLH-982984B

| TYPICAL PROPERTIES | |
|---|---|
| COLOR | Light green with gold sparkle |

(continued)

| TYPICAL PROPERTIES | |
| --- | --- |
| TEXTURE | Light gel |
| IDENTITY | Gel |
| VISCOSITY | 1,400 ± 200 cps |
| pH | 4.50 ± 0.50 |

SUPPLIERS

1. ISP
2. Rohm and Haas Company, Inc.
3. **Engelhard Corporation**
4. Shaw Mudge & Company

**Low pH Shampoo**

CLT-983049-1A

| PHASE | INGREDIENTS | | %WT. |
| --- | --- | --- | --- |
| A. | Water | (q.s. to 100%) | 67.47 |
| | Celquat H-100 (Polyquaternium-4)[1] | | 0.50 |
| | Structure Plus (Acrylates/Aminoacrylates Copolymer)[1] | | 8.00 |
| B. | Sodium Laureth Sulfate (jeelate ES-270)[2] | | 15.00 |
| | Cocamidopropyl Betaine (jeeteric CAB-LC)[2] | | 4.00 |
| | Linoleamidopropyl PG-Dimonium Chloride Phosphate Dimethicone (Monasil PLN)[3] | | 1.00 |
| C. | Citric Acid (25% aqueous solution) | | 3.50 |
| | Fragrance (16148M)[4] | | 0.40 |
| | Methylchloroisothiazolinone (and) Methylisothiazolinone (Kathon CG)[5] | | 0.05 |
| | Cloisonné® Sparkle Gold 222J (Mica (and) Titanium Dioxide (and) Iron Oxides)[6] | | 0.08 |
| | | | 100.00 |

**PROCEDURE**

I. Using moderate propeller agitation, add Celquat from Phase A with mixing at room temperature. Heat to 50°C and add Structure Plus with mixing.

II. Add Phase B ingredients in the order listed at 50°C with mixing. Avoid aeration.

III. Cool down to 40°C and add Phase C ingredients one by one. Stop mixing at 35°C.

**Low pH Shampoo**

CLT-983049-1A

| TYPICAL PROPERTIES | |
|---|---|
| COLOR | Gold |
| TEXTURE | Light gel |
| IDENTITY | Liquid soap |
| VISCOSITY | $8,000 \pm 1,000$ cps |
| pH | $4.50 \pm 0.50$ |

SUPPLIERS

1. National Starch & Chemical Company
2. Jeen International Corporation
3. Uniqema
4. Shaw Mudge & Company
5. Rohm and Haas Company, Inc.
6. **Engelhard Corporation**

**Conditioner**

CLH-983050

| PHASE | INGREDIENTS | | %WT. |
|---|---|---|---|
| A. | Water | (q.s. to 100%) | 88.60 |
| | Cetearyl Alcohol (and) PEG-40 Hydrogenated Castor Oil (and) Stearalkonium Chloride (Jeequat ASP)[1] | | 8.00 |
| | Ceteareth-20 (and) Cetearyl Alcohol (Jeecol CS-20D)[1] | | 2.00 |
| B. | Butyfparaben (and) Iodopropynyl Butylcarbamate (and) Propylene Glycol (Jeecide Butyl Plus)[1] | | 1.00 |
| | Fragrance (16149A)[2] | | 0.20 |
| | Cloisonné® Sparkle Gold 222J (Mica (and) Titanium Dioxide (and) Iron Oxides)[3] | | 0.20 |
| | | | 100.00 |

**PROCEDURE**

I. Disperse ingredients of Phase A in water, heat to 70°C and mix until uniform.

II. Cool to 35°C and add ingredients of Phase B one by one. Mix until uniform.

**Conditioner**

CLH-983050

| TYPICAL PROPERTIES | |
|---|---|
| COLOR | Gold |
| TEXTURE | Light cream |
| IDENTITY | Oil/water emulsion |
| VISCOSITY | $9,000 \pm 500$ cps |
| pH | $5.50 \pm 0.50$ |

SUPPLIERS

1. Jeen International Corporation
2. Shaw Mudge & Company
3. **Engelhard Corporation**

**Highlighting Hair Gel**

CLH-921237

| PHASE | INGREDIENTS | | %WT. |
|---|---|---|---|
| A. | Carbomer (Acritamer 940)[1] (2% Aqueous Solution) | | 40.00 |
| | Methyl Gluceth-20 (Glucam E-20)[2] | | 2.00 |
| | Propylene Glycol | | 2.00 |
| | Glycerin | | 1.00 |
| | Panthenol (DL-Panthenol)[3] | | 1.00 |
| B. | Water | | 2.00 |
| | Triethanolamine | | 1.00 |
| C. | Water | (q.s. to 100%) | 28.90 |
| | Sodium Hexametaphosphate | | 0.10 |
| | Preservatives | | q.s. |
| D. | Cloisonné® Gold 222C (Mica (and) Titanium Dioxide (and) Iron Oxides)[4] | | 5.00 |
| | SDA Alcohol 40 | | 15.00 |
| | PVP (PVP K-30)[5] | | 2.00 |
| | | | 100.00 |

PROCEDURE

I. Mix all the ingredients in Phase A in a suitable vessel until completely uniform.

II. Add premixed Phase B to Phase A while mixing until completely uniform.

III. In a separate vessel, combine all ingredients in Phase C.

IV. Add Phase C to Phase A-B and mix until uniform.

V. Add premixed Phase D to Phase A-B-C and mix until uniform.

**Highlighting Hair Gel**

CLH-921237

| TYPICAL PROPERTIES | |
|---|---|
| COLOR | Gold |
| ODOR | Characteristic |
| APPEARANCE | Luminous |
| VISCOSITY | $732,000 \pm 70,000$ cps |
| pH | $6.5, \pm 0.5$ |
| SPECIFIC GRAVITY | $0.99 \pm 0.10$ |

SUPPLIERS

1. R.I.T.A. Corporation
2. Amerchol Corporation
3. Tri-K Industries, Inc.
4. **Engelhard Corporation**
5. ISP

**Bath & Shower Gel**

CLT-960268M

| PHASE | INGREDIENTS | | %WT. |
|---|---|---|---|
| A. | Water | (q.s. to 100%) | 67.80 |
| | Acryfates/C10-30 Alkyl Acrylate Crosspolymer (Carbopol ETD-2020)[1] | | 0.70 |
| | Preservatives | | q.s. |
| | Propylene Glycol | | 1.00 |
| | Cloisonné® Sparkle Gold 222J (Mica (and) Titanium Dioxide (and) Iron Oxides)[2] | | 0.10 |
| B. | TEA-Lauryl Sulfate (Standapol T)[3] | | 23.00 |
| | Cocamidopropyl Betaine (Tego Betaine F-50)[4] | | 3.50 |
| | Cocamide DEA (Standamid SD)[3] | | 3.00 |
| | Disodium EDTA | | 0.05 |
| C. | Fragrance (Fruity Floral TF-157146)[5] | | 0.20 |
| | Red 40 (0.5% aqueous solution) | | 0.45 |
| D. | Triethanolamine (TEA-99%)[6] | | 0.20 |

(continued)

| PHASE | INGREDIENTS | %WT. |
|-------|-------------|------|
| | | 100.00 |

## PROCEDURE

I. Disperse Carbopol ETD-2020 in water in a suitable vessel with constant agitation. Mix until uniform and add the rest of the ingredients.
II. Heat to 65°C and add one by one Phase B ingredients with continuing mixing.
III. Cool down to 40°C and add Phase C and Phase D.

**Bath & Shower Gel**

CLT-960268M

| TYPICAL PROPERTIES | |
|--------------------|---|
| COLOR | Peach with gold sparkle |
| ODOR | Characteristic |
| APPEARANCE | Lustrous |
| VISCOSITY | 2,044 ± 200 cps |
| pH | 6.2 ± 0.5 |
| SPECIFIC GRAVITY | 1.0 ± 0.1 |

SUPPLIERS

1. BF Goodrich Specialty Chemicals
2. **Engelhard Corporation**
3. Henkel Corporation
4. Goldschmidt Chemical Corporation
5. Technology, Flavors & Fragrances, Inc.
6. Union Carbide Corporation

**Bath Dusting Powder**

CLT-930169

| PHASE | INGREDIENTS | | %WT. |
|-------|-------------|---|------|
| A. | Mearltalc® TCA (Talc (and) Lauroyl Lysine)[1] | (q.s. to 100%) | 84.00 |
| | Magnesium Carbonate (Magnesium Carbonate, Light USP 309)[2] | | 4.50 |
| | Coslin™ C-100 (Kaolin)[1] | | 3.00 |
| | Zinc Stearate | | 2.50 |
| | Zinc Oxide (Zinc Oxide USP 66)[2] | | 2.00 |
| | Fragrance | | 1.00 |

(continued)

| PHASE | INGREDIENTS | %WT. |
|---|---|---|
| | Timica® Silver Sparkle 5500 (Mica (and) Titanium Dioxide)[1] | 3.00 |
| | Preservatives | q.s. |
| | | 100.00 |

## PROCEDURE

I. Premix a dry master batch of perfume oil and Magnesium Carbonate.
II. Mix with the rest of ingredients until uniform.

**Bath Dusting Powder**

CLT-930169

| TYPICAL PROPERTIES | |
|---|---|
| COLOR | White |
| ODOR | Characteristic |
| APPEARANCE | Satiny |
| TEXTURE | Smooth |
| BULK DENSITY | $12.8 \pm 0.5$ lbs/ft$^3$ |
| COVERAGE | Very good |
| SKIN ADHESION | Very good |

SUPPLIERS

1. **Engeihard Corporation**
2. Whittaker, Clark & Daniels, Inc.

**Liquid Pearl Bath Soap**

CLT-920053

| PHASE | INGREDIENTS | | %WT. |
|---|---|---|---|
| A. | Water | (q.s. to 100%) | 64,20 |
| | Xanthan Gum (Keitrol T)[1] | | 0.60 |
| | TEA-Lauryl Sulfate (Standapol T)[2] | | 35.00 |
| | Flamenco® Superpearl 120C (Mica (and) Titanium Dioxide)[3] | | 0.20 |
| | Preservatives, Fragrance and Color | | q.s. |
| | | | 100.00 |

PROCEDURE

I. Disperse Xanthan Gum in water.

II. Add TEA-Lauryl Sulfate with stirring.

III. Continue stirring and add Flamenco Superpearl 120C.

IV. When well dispersed, add other ingredients and continue to stir until uniform.

**Liquid Pearl Bath Soap**

CLT-920055

| TYPICAL PROPERTIES | |
| --- | --- |
| COLOR | Eggwhite |
| ODOR | Characteristic |
| APPEARANCE | Pearlescent |
| VISCOSITY | 340 ± 30 cps |
| pH | 7.1 ± 0.5 |
| SPECIFIC GRAVITY | 1.0 ± 0.10 |

SUPPLIERS

1. The NutraSweet Kelco Company
2. Henkel Corporation
3. **Engelhard Corporation**

**Skin Moisturizing Gel**

CLT-970039M

| PHASE | INGREDIENTS | | %WT. |
| --- | --- | --- | --- |
| A. | Water | (q.s. to 100%) | 72.00 |
| | Preservatives | | q.s. |
| | Carbomer (Carbopol 941)[1] | | 0.25 |
| B. | 1, 3 Butylene Glycol | | 8.00 |
| | Flamenco® Gold 220C (Mica (and) Titanium Dioxide)[2] | | 0.30 |
| C. | Glyceryl Polyacrylate (and) Glycerine (and) | | |
| | Water (Hispagel 200)[3] | | 15.00 |
| | Squalane (Fitoderm)[5] | | 2.00 |
| D. | Water | | 2.00 |
| | Triethanolamine | | 0.25 |

(continued)

| PHASE | INGREDIENTS | %WT. |
|-------|-------------|------|
| E. | Fragrance (Flowering Herb TF-576631)[4] | 0.20 |
| | | $\overline{100.00}$ |

## PROCEDURE

I. Using moderate propeller agitation, dissolve preservatives in water. Then sprinkle the Carbomer into the water and allow to mix until fully hydrated.

II. Add Phase B to Phase A.

III. In a separate vessel weigh out the Hispagel 200. Add Phase A-B ingredients to the Hispagel 200 with gentle mixing. Add the rest of Phase C ingredients to Phase A-B.

IV. Add premixed Phase D to Phase A-B-C.

V. Add Phase E to Phase A-B-C-D.

**Skin Moisturizing Gel**

CLT-970039M

| TYPICAL PROPERTIES | |
|--------------------|--|
| COLOR | Ivory with gold sparkle |
| ODOR | Characteristic |
| APPEARANCE | Lustrous |
| VISCOSITY | 32,190 ± 3,000 cps |
| pH | 5.4, ± 0.5 |
| SPECIFIC GRAVITY | 1.0 ± 0.1 |

SUPPLIERS

1. BF Goodrich Specialty Chemicals
2. **Engelhard Corporation**
3. Hispano Quimica S.A./Centerchem, Inc.
4. Technology, Flavors & Fragrances, Inc.

**Sunshine Body Cream**

CLC-983005F

| PHASE | INGREDIENTS | | %WT. |
|-------|-------------|--|------|
| A. | Water | (q.s. to 100%) | 70.70 |

(continued)

| PHASE | INGREDIENTS | %WT. |
|---|---|---|
| | Propylene Glycol | 2.50 |
| | Triethanolamine | 1.00 |
| | Disodium EDTA | 0.05 |
| | Preservatives (water soluble) | q.s. |
| B. | Stearic Acid (Emersol 132)[1] | 4.00 |
| | Isopropyl Palmitate (Jeechem IPP)[2] | 4.00 |
| | Glyceryl Stearate SE (Cerasynt Q)[3] | 4.00 |
| | Glyceryl Stearate (Cerasynt SD)[3] | 3.00 |
| | Dimethicone (Dow Corning 200 Fluid)[4] | 0.50 |
| | Acetylated Lanolin (Modulan)[5] | 0.50 |
| | Cetyl Alcohol (Crodacol C-95)[6] | 1.00 |
| | Mineral Oil | 5.50 |
| | Preservatives (oil soluble) | q.s. |
| C. | Water | 1.00 |
| | Preservatives (water soluble) | q.s. |
| | Chamomile (Anthemis Nobilis) Extract (Chamomile Extract 43558)[7] | 0.50 |
| D. | Mineral Oil | 1.50 |
| | Cellini® Yellow 220CY5F (Mica (and) Titanium Dioxide (and) Yellow 5 Lake (and) Hydrogenated Polyisobutene (and) Palmitic Acid (and) Methylparaben (and) Propylparaben (and) Butyiparaben)' | 0.25 |
| | | 100.00 |

**SunShine Body Cream**

CLC-983005F

**PROCEDURE**

I. Add ingredients of Phase A in the order listed and heat to 75°C with mixing.

II. Heat Phase B at 75°C and add it to Phase A with mixing.

III. Premix the ingredients of Phase C and add them to Phase A-B at 45°C.

IV. Premix ingredients of Phase D and add them to the batch at 40°C. Stop mixing at 35°C.

| TYPICAL PROPERTIES | |
|---|---|
| COLOR | Bright yellow |
| TEXTURE | Light cream |
| IDENTITY | Oil in water emulsion |
| VISCOSITY | 150,000 ± 5,000 cps |
| pH | 7.50 ± 0.50 |

SUPPLIERS

1. Henkel Corporation

2. Jeen International Corporation

3. ISP

4. Dow Corning Corporation

5. Amercol Corporation

6. Croda, Inc.

7. Dr. Madis Laboratories, Inc.

8. **Engelhard Corporation**

**Silky BHA Lotion**

CLC-983027B

| PHASE | INGREDIENTS | | %WT. |
|---|---|---|---|
| A. | Water | (q.s. to 100%) | 72.95 |
| | Isodecyl Octanoate (Jeechem IDO)[7] | | 8.00 |
| | Disodium EDTA | | 0.05 |
| B. | Poiyacryiamide (and) C13-14 Isoparaffin (and) | | |
| | Laureth-7 (Sepigel 305)[2] | | 5.50 |
| C. | Saiicylic Acid (and) Butylene Glycol (and) Acacia Senegal | | |
| | Extract (Liquid Beta-Hydroxyde)[3] | | 8.00 |
| | Oat Beta Glucan (Tech-O 6-070 L)[4] | | 0.50 |
| D. | Chamomile (Anthemis Nobilis) Oil[5] | | 2.00 |
| | Mearlmica® SVA (Mica (and) Lauroyl Lysine)[6] | | 2.00 |
| E. | Preservatives | | q.s. |
| | Fragrance (16148M)[7] | | 0.20 |
| | Red 4 (0.1% aqueous solution) | | 0.30 |
| | Flamenco® Satin Pearl 3500 (Mica (and) Titanium Dioxide)[6] | | 0.50 |
| | | | 100.00 |

**PROCEDURE**

I. Mix ingredients of Phase A at room temperature. Heat to 60°C and add Phase B. Mix until uniform.

II. Cool to 45°C and add Phase C ingredients one by one with mixing.

III. Add the premixed Phase D ingredients.

IV. Add Phase E and mix until uniform.

**Silky BHA Lotion**

CLC-983027B

| TYPICAL PROPERTIES | |
|---|---|
| COLOR | Pearly pink |
| TEXTURE | Light lotion |
| IDENTITY | Oil in water emulsion |
| VISCOSITY | 19,000 ± 2,000 cps |
| pH | 5.00 ± 0.50 |

SUPPLIERS

1. Jeen International Corporation
2. Seppic
3. Bioetica, Inc.
4. Beacon CMP Corporation
5. Dr. Madis Laboratories, Inc.
6. **Engelhard Corporation**
7. Shaw Mudge & Company

**Cleansing Scrub**

CLT-983052A

| PHASE | INGREDIENTS | | %WT. |
|---|---|---|---|
| A. | Water | (q.s. to 100%) | 82.51 |
| | Acrylates/C10-30 Alkyl Acrylate Crosspolymer (Carbopol ETD 2020)[1] | | 1.00 |
| B. | TEA-Lauryl Sulfate (Jeelate TLS-40)[2] | | 15.00 |
| | Jojoba Esters (Rovibeads Blue 28/60)[1] | | 0.40 |
| | Fragrance (16149A)[4] | | 0.25 |
| | Preservatives | | q.s. |
| | Flamenco® Ultra Sparkle 4500 (Mica (and) Titanium Dioxide)[5] | | 0.04 |
| | Triethanolamine | | 0.80 |
| | | | 100.00 |

**PROCEDURE**

I. Disperse Carbopol in water with high-shear agitation. Mix until uniform.
II. Add ingredients of Phase B one by one and mix until uniform.

**Cleansing Scrub**

CLT-985052A

| TYPICAL PROPERTIES | |
|---|---|
| COLOR | Clear with silver sparkles and blue beads |
| TEXTURE | Light gel |
| IDENTITY | Gel |
| VISCOSITY | 20,000 ± 500 cps |
| pH | 5.50 ± 0.50 |

SUPPLIERS

1. BF Goodrich Specialty Chemicals
2. Jeen International Corporation
3. ROVI GmbH
4. Shaw Mudge & Company
5. **Engeihard Corporation**

**Snow White Body Cream**

CLC-983024

| PHASE | INGREDIENTS | | %WT. |
|---|---|---|---|
| A. | Water | (q.s. to 100%) | 68.95 |
| | Propylene Glycol | | 2.50 |
| | Triethanolamine | | 1.00 |
| | Disodium EDTA | | 0.05 |
| | Preservatives | | q.s. |
| B. | Stearic Acid (Emersol 132)[1] | | 4.00 |
| | Isopropyl Palmitate (jeechem IPP)[2] | | 4.00 |
| | Glyceryl Stearate SE (Cerasynt Q)[3] | | 4.00 |
| | Glyceryl Stearate (Cerasynt SD)[3] | | 3.00 |
| | Dimethicone (Dow Corning 200 Fluid)[4] | | 0.50 |
| | Acetylated Lanolin (Modulan)[5] | | 0.50 |
| | Cetyl Alcohol (Crodacol C-95)[6] | | 1.00 |
| | Mineral Oil | | 7.00 |
| | Preservatives (oil soluble) | | q.s. |
| C. | Water | | 1.00 |
| | Preservatives (water soluble) | | q.5. |
| | Chamomile (Anthemis Nobilis) Extract (Chamomile Extract 43338)[7] | | 0.50 |
| D. | Polysorbate 80 | | 0.40 |
| | Biju® Ultra UFC (Bismuth Oxychloride)[8] | | 1.60 |
| | | | 100.00 |

## PROCEDURE

I. Add ingredients of Phase A in the order listed and heat to 75°C with mixing.

II. Heat Phase B at 75°C and add it to Phase A with mixing.

III. Premix the ingredients of Phase C and add them to Phase A-B at 45°C

IV. Premix ingredients of Phase D and add them to the batch at 40°C. Stop mixing at 35°C.

**Snow White Body Cream**

CLC-983024

| TYPICAL PROPERTIES | |
|---|---|
| COLOR | Bright white |
| TEXTURE | Light cream |
| IDENTITY | Oil in water emulsion |
| VISCOSITY | 130,000 ± 5,000 cps |
| pH | 7.50 ± 0.50 |

SUPPLIERS

1. Henkel Corporation
2. Jeen International Corporation
3. ISP
4. Dow Corning Corporation
5. Amerchol Corporation
6. Croda, Inc.
7. Dr. Madis Laboratories, Inc.
8. **Engelhard Corporation**

**Clarifying Bright Lotion**

CLC-983035A

| PHASE | INGREDIENTS | | %WT. |
|---|---|---|---|
| A. | Water | (q.s. to 100%) | 75.50 |
| | Hydroxyethylcellulose (Natrosol 250HR)[1] | | 0.50 |
| | Glycerin | | 1.00 |
| | PEG-40 Stearate (Myrj 52)[2] | | 1.00 |
| | Stearamidopropyl PG-Dimonium Chloride Phosphate (and) | | |
| | Cetyl Alchol (Phospholipid SV)[2] | | 2.00 |
| | Disodium EDTA | | 0.10 |
| | Cloisonné® Sparkle Bronze 250J (Mica (and) Iron Oxides)[3] | | 0.30 |

(continued)

| PHASE | INGREDIENTS | %WT. |
|---|---|---|
| B. | Isostearyl Paimitate (jeechem ISP)[4] | 3.00 |
| | Petrolatum | 3.00 |
| | Octyl Methoxycinnamate (Parsol MCX)[5] | 3.00 |
| | Ceteareth 20 (and) Cetearyl Alcohol (Jeecol CS-20D)[4] | 1.00 |
| | Steareth-2 (Brij 72)[2] | 1.00 |
| | BHT | 0.05 |
| C. | Polyquaternium-37 (and) Propylene Glycol/Dicaprylate Dicaprate (and) PPG-1 Trideceth-6 (Salcare SC 96)[6] | 2.75 |
| D. | Water | 2.00 |
| | Preservatives | q.s. |
| | Sodium Metabisulfite | 0.10 |
| | Fragrance (16148M)[7] | 0.20 |
| | Bearberry (Arctostaphylos UVA Ursi) Extract (Malfade)[8] | 3.50 |
| | | 100.00 |

## PROCEDURE

I. Using moderate propeller agitation, disperse Natrosol 250HR in water and heat to 70°C. Add the rest of the ingredients with mixing.
II. Heat Phase B ingredients to 70°C and add them to Phase A.
III. Cool to 40°C and add Phase C. Mix until uniform.
IV. At 35°C add Phase D ingredients one by one. Mix until uniform.

**Clarifying Bright Lotion**

CLC-983035A

| TYPICAL PROPERTIES | |
|---|---|
| COLOR | Tan with gold sparkle |
| TEXTURE | Lotion |
| IDENTITY | Oil in water emulsion |
| VISCOSITY | 22,000 2,000 cps |
| pH | 5.00 ± 0.50 |

SUPPLIERS

1. Aqualon
2. Uniqema
3. **Engelhard Corporation**
4. Jeen International Corporation
5. Roche Vitamins and Fine Chemicals
6. CIBA Specialty Chemicals
7. Shaw Mudge & Company

8. Hispano Quimica S.A./Centerchem, Inc.

**Moisturizing Body Veil**

CLT-973014C

| PHASE | INGREDIENTS | | %WT. |
|---|---|---|---|
| A. | Water | (q.s. to 100%) | 92.35 |
| | Acrylates/Steareth-20 Methacrylate Copolymer (Aculyn 22)[1] | | 3.00 |
| | Acrylates Copolymer (Salcare 5C81)[2] | | 0.80 |
| | Fragrance (16149A)[3] | | 0.20 |
| | Disodium EDTA | | 0.05 |
| B. | Triethanolamine | | 1.00 |
| C. | Water | | 1.00 |
| | Preservatives | | q.s. |
| | Aloe Barbadensis Gel (Aloe Vera Concentrate 21511-000)[4] | | 1.00 |
| | Chamomile (Anthemis Nobilis) Extract[4] | | 0.50 |
| D. | Flamenco® Sparkle Orange 320J (Mica (and) Titanium Dioxide)[5] | | G.10 |
| | | | 100.00 |

PROCEDURE

I. Using moderate propeller agitation, add ingredients of Phase A in the order listed, at room temperature.

II. Add Triethanolamine and mix until the gel is formed.

III. Premix the ingredients of Phase C and add them to the kettle. Add Phase D with gentle agitation.

**Moisturizing Body Veil**

CLT-973014C

| TYPICAL PROPERTIES | |
|---|---|
| COLOR | Translucent ge! with orange sparkles |
| TEXTURE | Silky gel |
| IDENTITY | Gel |
| VISCOSITY | 280,000 ± 5,000 cps |
| pH | 7.50 ± 0,50 |

SUPPLIERS

1. ISP
2. CIBA Specialty Chemicals

3. Shaw Mudge & Company
4. Dr. Madis Laboratories, Inc.
5. **Engelhard Corporation**

**Face Mask For Oily Skin**

CLF-973015M

| PHASE | INGREDIENTS | | %WT. |
|---|---|---|---|
| A. | Water | (q.s. to 100%) | 74.65 |
| | Magnesium Aluminum Silicate (Veegum)[1] | | 3.50 |
| | Xanthan Gum (Keltrol T)[2] | | 1.00 |
| | Glycerin | | 5.00 |
| | Preservatives (water soluble) | | q.s. |
| B. | Coslin™ C-100 (Kaolin)[3] | | 4.00 |
| C. | Isostearyl Palmitate (Jeechem ISP)[4] | | 3.00 |
| | Sorbitan Stearate (Arlacel 60)[5] | | 1.60 |
| | Beeswax (Beeswax White Refined Bleached N. F.)[6] | | 2.00 |
| | Refined Milk Lipids (Monalac ML)[7] | | 1.00 |
| | Oat (Avena Sativa) Flour | | 1.00 |
| | Preservatives (oil soluble) | | q.s. |
| D. | Water | | 1.00 |
| | Preservatives (water soluble) | | q.s. |
| E. | Water | | 1.50 |
| | Flamenco® Ultra Silk 2500 (Mica (and) Titanium Dioxide)[3] | | 0.50 |
| | Fragrance (16148 M)[8] | | 0.25 |
| | | | 100.00 |

**PROCEDURE**

I. Using moderate propeller agitation, add ingredients of Phase A in the order listed at room temperature and mix until uniform. Heat to 75°C with mixing.

II. Add Phase B at 75°C and homogenize for 15 minutes.

III. Premix the ingredients of Phase C and heat to 75°C with mixing.

IV. Add Phase C to A and B and mix until uniform using propeller agitation.

V. Cool down to 45°C and add Phase D and E. Stop mixing at 35°C.

**Face Mask For oily Skin**

CLF-973015M

| TYPICAL PROPERTIES | |
|---|---|
| COLOR | Off-white |

(continued)

| TYPICAL PROPERTIES | |
|---|---|
| TEXTURE | Light cream |
| IDENTITY | Oil in water emulsion |
| VISCOSITY | 50,000 ± 3,000 cps |
| pH | 7.00 ± 0.50 |

SUPPLIERS

1. R. T. Vanderbilt Company, Inc.
2. The NutraSweet Kelco Company
3. **Engelhard Corporation**
4. Jeen International Corporation
5. ICI Surfactants
6. Frank B. Ross Co., Inc.
7. Uniqema
8. Shaw Mudge & Company

**Icy Get Toothpaste**

CLT-992001A

| PHASE | INGREDIENTS | %WT. | |
|---|---|---|---|
| A. | Water | (q.s. to 100%) | 6.25 |
| | Sorbitol (Liponic 70-NC)[1] | | 54.30 |
| | Glycerine | | 10.00 |
| | Cellulose Gum | | 0.50 |
| | PEG-32 (Carbowax PEG 1450)[2] | | 3.00 |
| B. | Water | | 5.00 |
| | Sodium Benzoate | | 0.30 |
| | Sodium Saccharin | | 0.20 |
| C. | Hydrated Silica (Zeodent 165)[3] | | 5.00 |
| | Hydrated Silica (Zeodent 113)[3] | | 15.00 |
| D. | Flavor (Sweet Mint # 26037G)[4] | | 0.20 |
| | Blue 1 (0.5% aqueous solution) | | 0.10 |
| | Flamenco® Sparkle Blue 620J (Mica (and) Titanium Dioxide)[5] | | 0.15 |
| | | | 100.00 |

**PROCEDURE**

I. Premix water, Sorbitol and Glycerin, and disperse Cellulose Gum with mixing until thoroughly dispersed.

II. Add PEG-32 and heat to 50°C.

III. At 40°C add premixed Phase B to Phase A while mixing until completely uniform.

IV. Add Phase C to Phase A-B while under agitation.

V. Add Phase D ingredients one by one to Phase A-B-C mixing until completely uniform.

**Icy Gel Toothpaste**

CLT-992001A

| TYPICAL PROPERTIES | |
|---|---|
| COLOR | Blue with blue sparkles |
| ODOR | Mint |
| TEXTURE | Viscous gel |
| IDENTITY | Gel |
| VISCOSITY | 47,000 ± 5,000 cps |
| pH | 6.5 ± 0.50 |

SUPPLIERS

1. Lipo Chemicals, Inc.
2. Dow Chemical Company
3. J. M. Huber Corporation
4. Shaw Mudge & Company
5. **Engelhard Corporation**

**Pearly Bronze/Copper Suntan Cream**

CLS-921965

| PHASE | INGREDIENTS | | %WT. |
|---|---|---|---|
| A. | Octyl Methoxycinnamate (Parsol MCX)[1] | | 3.00 |
| | Lanolin Acid (Amerlate LFA)[2] | | 3.00 |
| | Isopropyl Lanolate (Ameriate P)[2] | | 1.50 |
| | Petrolatum (and) Lanolin Alcohol (Amerchol CAB)[2] | | 4.00 |
| | Mineral Oil (Carnation White Mineral Oil)[3] | | 3.50 |
| | Glyceryl Stearate SE (Tegin G)[4] | | 6.00 |
| | Stearyl Alcohol | | 3.00 |
| | Ozokerite (Ozokerite Wax 170D)[5] | | 5.00 |
| | Preservatives (oil soluble) | | q.s. |
| | Antioxidants | | q.s. |
| B. | Triethanolamine | | 1.00 |
| | Methyl Gluceth-10 (Glucam E-10)[2] | | .2.50 |
| | Water | (q.s. to 100%) | 62.50 |
| | Preservatives (water soluble) | | q.s. |
| | Fragrance | | q.s. |
| C. | Cloisonné® Super Copper 350Z (Mica (and) Iron Oxides)[6] | | 5.00 |

(continued)

| PHASE | INGREDIENTS | %WT. |
|---|---|---|
| | | 100.00 |

## PROCEDURE

I. Separately heat Phase A and B to 80° ± 3°C.

II. Stir Phase A into Phase B until homogeneous. Then cool to 40°C with slow stirring.

III. Disperse pigment (Phase C) into Phase A-B.

IV. Cool to 30°C and fill.

**Pearly Bronze/Copper Suntan Cream**

CLS-921965

| TYPICAL PROPERTIES | |
|---|---|
| COLOR | Copper |
| ODOR | Characteristic |
| APPEARANCE | Pearly |
| TEXTURE | Creamy |
| IDENTITY | Oil/water emulsion |
| VISCOSITY | 22,000 ± 200 cps |
| pH | 7,5 ± 0.5 |
| SPECIFIC GRAVITY | 0.93 ± 0.10 |

SUPPLIERS

1 Roche Vitamins and Fine Chemicals
2. Amerchol Corporation
3. Witco Corporation
4. Goldschmidt Chemical Corporation
5 Frank B. Ross Co., Inc.
6. **Engelhard Corporation**

**Sunscreen Cream**

CLS-910997A

SPF-13*

| PHASE | INGREDIENTS | | %WT. |
|---|---|---|---|
| A. | PVP/Eicosene Copolymer (Ganex V-220)[1] | | 4.12 |
| | Stearic Acid | | 3.50 |
| | Triisostearyl Trilinoleate (Schercemol TIST)[2] | | 3.30 |
| | Isononyl Isononanoate (Dermol 99)[3] | | 3.50 |
| | Octyl Methoxycinnamate (Parsol MCX)[4] | | 2.62 |
| | Cetyl Alcohol (Crodacol C-95)[5] | | 1.00 |
| | Benzophenone-3 (Uvinul M-40)[6] | | 0.75 |
| | Dimethicone (Dow Corning 200 Fluid)[7] | | 0.22 |
| | Preservatives (oil soluble) | | q.s. |
| B. | DEA-Cetyl Phosphate (Amphisol)[4] | | 2.03 |
| | Water | (q.s. to 100%) | 48.94 |
| | Glycerin | | 4.12 |
| | Preservatives (water soluble) | | q.s. |
| | Carbomer 940 (2% aqueous solution)[8] | | 4.60 |
| C. | Triethanolamine | | 0.30 |
| | Water | | 1.00 |
| D. | Mearlmaid® AA (Water (and) Guanine (and) Isopropyl Alcohol (and) Methyl Cellulose)[9] | | 10.00 |
| | Water | | 10.00 |
| | | | 100.00 |

**PROCEDURE**

I. Separately heat Phase A and Phase C to 80° ± 3°c while mixing until completely uniform.

II. Stir Phase A into Phase B until homogeneous.

*(Procedure continued on back.)*

\*   As determined by an independent laboratory based on a 5 subject screening panel.

**Sunscreen Cream**

CLS-910997A

SPF 13*

PROCEDURE, *CONTINUED*

III. Add premixed Phase C To Phase A-B and then cool to 40°C with slow stirring.

IV. Add Phase D with stirring.

V. Cool to 30°C and fill.

| TYPICAL PROPERTIES | |
|---|---|
| COLOR | White |
| ODOR | Characteristic |
| APPEARANCE | Pearlescent |
| TEXTURE | Creamy |
| IDENTITY | Oil/water emulsion |
| VISCOSITY | 10,850 ± 1,000 cps |
| pH | 6.7 ± 0.5 |
| SPECIFIC GRAVITY | 0.98 ± 0.10 |

SUPPLIERS

1. ISP

2. Scher Chemicals, Inc.

3. Alzo International, Inc.

4. Roche Vitamins and Fine Chemicals

5. Croda, Inc.

6. BASF Corporation

7. Dow Corning Corporation

8. BF Goodrich Specialty Chemicals

9. **Engelhard Corporation**

**Bronze Self-Tanning Cream**

CLC-983036A

| PHASE | INGREDIENTS | | %WT. |
|---|---|---|---|
| A. | Water | (q.s. to 100%) | 71.05 |
| | Stearamidopropyl PG-Dimonium Chloride Phosphate (and) Cetyl Alcohol (Phospholipid SV)[7] | | 2.00 |
| | Preservatives (water soluble) | | 0.5. |
| | Cloisonné® Sparkle Copper 350J (Mica (and) Iron Oxides)[2] | | 0.30 |
| | Cloisonne Satin Bronze 250 (Mica (and) Iron Oxides)[2] | | 0.30 |
| B. | Isostearyl Palmitate (Jeechem ISP)[3] | | 1.00 |
| | Petrolatum | | 3.00 |
| | Ceteareth 20 (and) Cetearyl Alcohol (Jeecol CS-20D)[3] | | 1.00 |
| | Preservatives (oil soluble) | | q.s. |
| | BHT | | 0.05 |
| C. | Polyquaternium-37 (and) Propylene Glycol/Dicaprylate Dicaprate (and) PPG-1 Trideceth-6 (Salcare SC96)[4] | | 4.00 |
| D. | Water | | 9.00 |
| | Propyiene Glycol | | 4.00 |
| | Sodium Metabisulfite | | 0.10 |
| | Dihydroxyacetone[3] | | 4.00 |
| | Fragrance (16148M)[5] | | 0.20 |
| | | | 100.00 |

## PROCEDURE

I. Using moderate propeller agitation, add Phospholipid SV in water and heat to 70°C. Add the rest of the ingredients with mixing.

II. Heat Phase B ingredients to 70°C and add them to Phase A.

III. Cool to 40°C and add Phase C. Mix until uniform.

IV. Add the premixed Phase D with mixing. Stop mixing at 35°C.

**Bronze Self-Tanning Cream**

CLC-983036A

| TYPICAL PROPERTIES | |
|---|---|
| COLOR | Tan with gold sparkle |
| TEXTURE | Light cream |
| IDENTITY | Oil in water emulsion |
| VISCOSITY | 44,000 ± 2,000 cps |

(continued)

| TYPICAL PROPERTIES | |
|---|---|
| pH | 4.00 ± 0.50 |

SUPPLIERS

1. Uniqema
2. **Engelhard Corporation**
3. Jeen International Corporation
4. CIBA Specialty Chemicals
5. Shaw Mudge & Company

**Pearlescent Waterproof Sun Creme**

CLS-921967

| PHASE | INGREDIENTS | | % WT. |
|---|---|---|---|
| A. | PVP/Eicosene Copolymer (Ganex V-220)[1] | | 4.10 |
| | Stearic Acid | | 3.30 |
| | Triisostearyl Trilinoleate (Schercemol TtST)[2] | | 3.30 |
| | Isononyl Isononanoate (Dermol 99)[3] | | 3.30 |
| | Octyl Methoxycinnamate (Parsol MCX)[4] | | 2.60 |
| | Cetyl Alcohol (Crodacol C-95)[5] | | 0.80 |
| | Benzophenone-3 (Uvinul M-40)[1] | | 0.80 |
| | Dimethicone (Dow Corning 200 Fluid)[6] | | 0.20 |
| | Preservatives (oil soluble) | | q.s. |
| B. | DEA Cetyl Phosphate (Amphisol)[4] | | 2.00 |
| | Water | (q.s. to 100%) | 46.60 |
| | Glycerin | | 4.10 |
| | Preservatives (water soluble) | | q.s. |
| | Carbomer (Carbopol 940) (2% aqueous solution) | | 3.80 |
| C. | Triethanolamine | | 0.10 |
| D. | Flamenco® Ultra Silk 2500(Mica (and) Titanium Dioxode)[8] | | 25.00 |
| | | | 100.00 |

## PROCEDURE

I. Separately heat Phase A and Phase B to 80° ± 3°C while mixing until completely uniform.

II. Stir Phase A into Phase B until homogeneous.

III. Add Phase C to Phase A-B. Then cool to 40°C with slow stirring.

IV. Disperse pigment (Phase D) into warm base.

V. Cool to 30°C and fill.

**Pearlescent Waterproof Sun Creme**

CLS-921967

| TYPICAL PROPERTIES | |
|---|---|
| COLOR | White |
| ODOR | Characteristic |
| APPEARANCE | Pearlescent |
| TEXTURE | Creamy |
| IDENTITY | Oil/water emulsion |
| VISCOSITY | 28,500 ± 2,800 cps |
| pH | 6.8 ± 0.5 |
| SPECIFIC GRAVITY | 0.95 ± 0.10 |

SUPPLIERS

1. ISP
2. Scher Chemicals, Inc.
3. Alzo International, Inc.
4. Roche Vitamins and Fine Chemicals
5. Croda, Inc.
6. Dow Corning Corporation
7. BF Goodrich Specialty Chemicals
8. **Engelhard Corporation**

**Gel Cologne with Sparkles**

CLT-992032A

| PHASE | INGREDIENTS | | %WT. |
|---|---|---|---|
| A. | Water | (q.s. to 100%) | 34.87 |
| | Acrylates/C10-30 Alkyl Acrylate Crosspolymer | | |
| | (Carbopol ETD 2020)[1] | | 0.20 |
| | 2-Amino 2-Methyl Propanol (AMP-95)[2] | | 0.10 |
| B. | Fragrance (Amarige 19434V)[3] | | 4.00 |
| | Octoxynol-9 (Triton X-100 Surfactant)[4] | | 2.00 |
| | Butylene Glycol | | 1.00 |
| | PPG-20 Methyl Glucose Ether (Glucam P-20)[5] | | 1.00 |
| C. | SD Alcohol 40-1 Anhydrous | | 56.23 |
| | Flamenco® Ultra Sparkle 4500 (Mica (and) Titanium Dioxide)[6] | | 0.05 |
| | Green 5 (0.5% aqueous solution) | | 0.20 |
| | Ext. Red 4 (0.5% aqueous solution) | | 0.25 |
| | Blue 1 (0.5% aqueous solution) | | 0.10 |
| | | | 100.00 |

## PROCEDURE

I. Disperse Carbopol in water at room temperature and mix until uniform.
II. Add AMP with mixing.
III. Add premixed Phase B-C to Phase A and continue to mix.

## Gel Cologne with Sparkles

CLT-992032A

| TYPICAL PROPERTIES | |
| --- | --- |
| COLOR | Marble with sparkles |
| OOOR | Characteristic |
| APPEARANCE | Lustrous |
| pH | $6.0 \pm 0.5$ |
| SPECIFIC GRAVITY | $0.912 \pm 0.100$ |
| VISCOSITY | $530 \pm 50$ cps |

## SUPPLIERS

1. BF Goodrich Specialty Chemicals
2. Angus Chemical Company
3. Shaw Mudge & Company
4. Union Carbide
5. Amerchol Corporation
6. **Engelhard Corporation**

## Transparent Pearly Lotion

CLC-920048

| PHASE | INGREDIENTS | | %WT. |
| --- | --- | --- | --- |
| A. | Water | (q.s. to 100%) | 39.80 |
| B. | Alcohol SDA 40 | | 50.00 |
| | Fragrance | | 0.20 |
| C. | Mearlmaid® PLN (Water (and) Carbomer-940 (and) Diisopropanolamine (and) Guanine (and) Methylcellulose)[1] | | 10.00 / 100.00 |

## PROCEDURE

I. Add Phase A to Phase B.

II. Add combined Phases A and B to Phase C slowly with good, but not excessive agitation. (High speed agitation may cause fragmentation of the pearl essence crystals, with consequent decrease of luster.) Color may be introduced at any point as desired. Moisturizers, emollients and skin conditioners which do not reduce transparency may be added for special effects.

**Transparent Pearly Lotion**

CLC-920048

| TYPICAL PROPERTIES | |
|---|---|
| COLOR | White |
| ODOR | Characteristic |
| APPEARANCE | Pearly |
| VISCOSITY | $350 \pm 30$ cps |
| pH | $6.9 \pm 0.5$ |
| SPECIFIC GRAVITY | $0.92 \pm 0.10$ |

SUPPLIERS

1. **Engelhard Corporation**

**Claims**

**1.** Cosmetic formulation comprising 99,9 weight -% - 70 weigth-% of a basic cosmetic fomulation selected from the group of transparent pearly lotion, gel cologne with sparkles, pearlescent waterproof sun creme, bronze self tanning cream, sunscreen creme, pearl bronze/copper suntan cream, Icy gel toothpaste, face mask for oily skin, moisturizing body veil, clarifying bright lotion, snow white body cream, cleansing scrub, silky BHA lotion, sunshine body cream, skin moisturizing gel, liquid pearl bath soap, bath dusting powder, bath and shower gel, highlighting hair gel, conditioner, low pH shampoo, sparkle hair spray, festival hair gel, iridescent hair conditioner, conditioning shampoo, pearly foundation cream, blush stick, face bronze pressed powder, silky finish loose face powder, liquid makeup, body veil, earthtone makeup powder, liquid foundation, creamy peral blush, sheer leg makeup, face bronzer with sunscreen, silky face powder, shimmering pearl pressed powder blush, all purpose color stick, dual face powder, radiance pressed powder blush, gel blush frost, sparkling ruby nail enamel, poured eye shadow, cream eye shadow, emulsion cream mascera, eye shadow, liquid eyeliner, pressed powder eye shadow, emulsion cream eye shadow, charcoal silky powder eyeliner, loose powder eye shadow, concealer, sheer satin pressed powder eye shadows, crayon eye shadow, velvety pressed powder eye shadow, waterproof mascara, slender stick eye shadow, silky pressed powder eye shadow, lipstick, fair taupe lipstick, lip gloss with sunscreen, glossy lipstick, shimmering brick lipstick, lip pomade, soft lipstick, pressed lip powder, long wear lipstick, lipstick with sunscreen, lip glaze-formulation
and
0,01 - 30 weight-% glasses or color glass or glass ceramics or ceramics or glass powders or color glass powders or glass ceramic powders or ceramic powders or composite materials comprising glass or glass ceramics or ceramic powders or mixtures of glass, ceramic and glass ceramic powders.

**2.** Cosmetic composititon according to claim 1, wherein the basis. glass for the glasses or color glass or glass ceramics or ceramics or glass powders or color glass powders or glass ceramic powders or ceramic powders or composite materials comprising glass or glass ceramics or ceramic powders or mixtures of glass, ceramic and glass ceramic powders comprise advantageously the following components:

$SiO_2$ 0 - 80weight-%
$Na_2O$ 0 - 35weight-%

P$_2$O$_5$ 0 - 10 weight-%
MgO 0 - 20 weight-%
Ag$_2$O 0 - 2 weight-%
AgJ 0 - 0,5 weight-%
NaJ 0 - 5 weight-%
TiO$_2$ 0 - 5 weight-%
K$_2$O 0 - 35weight-%
ZnO 0 - 20 weight-%
Al$_2$O$_3$ 0 - 25 weight-%
B$_2$O$_3$ 0 - 25 weight-%

3. Cosmetic composition according to claim 1, wherein the particle size of the glass powder or the glass ceramic powder is < 100 μm, preferably < 50 μm, most preferable < 10 μm at most preferably < 1 μm.

4. Cosmetic composition, wherein the color glass comprises the following components:

SiO$_2$ 0 - 80 weight-%
Na$_2$O 0 - 35 weight-%
P$_2$O$_5$ 0 - 80 weight-%
MgO 0 - 5 weight-%
Ag$_2$O 0 - 0,5 weight-%
AgJ 0 - 0,5 weight-%
NaJ 0 - 5 weight-%
TiO$_2$ 0 - 5 weight-%
K$_2$O 0 - 35 weight-%
ZnO 0 - 10 weight-%
Al$_2$O$_3$ 0 - 25 weight-%
B$_2$O$_3$ 0 - 25 weight-%
SnO 0 - 5 weight-%
CeO$_2$ 0 - 3 weight-%
Au 0,001 - 0,1 weight-%